# EUROPEAN PATENT APPLICATION

(11) **EP 4 624 569 A1**
(43) Date of publication of application: **01.10.2025**
(21) Application number: 23893912.8
(22) Date of filing: 22.11.2023
(51) Int. Cl.: C12N 5/074, C12N 5/079, A61K 35/30, A61P 25/28, A61P 25/08, A61P 25/14, A61P 25/00, A61P 9/10

(54) **APPLICATION OF FOREBRAIN NEURAL PROGENITOR CELLS IN TREATMENT OF DISEASES ASSOCIATED WITH FOREBRAIN NEURAL CELL DEATH AND/OR INSUFFICIENCY**

(30) Priority: 22.11.2022 CN 202211466612
(71) Applicant: Zhejiang Huode Bioengineering Company Limited, Hangzhou, Zhejiang 310018 (CN)
(72) Inventor: FAN, Jing, Hangzhou, Zhejiang 310018 (CN); REN, Fang, Hangzhou, Zhejiang 310018 (CN); WANG, Anxin, Hangzhou, Zhejiang 310018 (CN); YAN, Yinggang, Hangzhou, Zhejiang 310018 (CN)
(74) Representative: Weickmann & Weickmann PartmbB
(86) International application number: PCT/CN2023/133378
(87) International publication number: WO 2024/109824

(57) **Abstract**

Provided is an application of a neural cell population in treating a nerve damage disease, a neurodegenerative disease, a neurodevelopmental disease, or another disorder associated with forebrain neural cell death and/or insufficiency. The neural cell population comprises forebrain neural progenitor cells (FNPC) having specific developmental features. Preferably, the forebrain neural progenitor cells having specific developmental features comprise high expressions of FOXG1, PAX6 and NESTIN markers. Preferably, the neural cell population is obtained by culturing human pluripotent stem cells (hPSC).

## Description

### CROSS-REFERENCE TO RELATED APPLICATIONS

This application is a National Stage Application and claims priority under 35 U.S.C. § 371 to Patent Cooperation Treaty application PCT/CN2023/133378, filed November 22, 2023, which claims the benefit of Chinese Patent Application No. 202211466612.0, filed November 22, 2022. Priority is claimed to these applications and the disclosures of these prior applications are considered part of the disclosure of this application and to the extent allowed the entire contents of the aforementioned applications are incorporated herein.

### CROSS-REFERENCE TO RELATED APPLICATIONS

This application claims priority to Chinese Patent Application No. 202211466612.0, titled "Application of Forebrain Neural Progenitor Cells in Treating Diseases Associated with Death and/or Dysfunction of Forebrain Neural Cells," filed on November 22, 2022. The entire contents of the aforementioned application are incorporated herein by reference.

### TECHNICAL FIELD

The present invention relates to the field of cell therapy in biotechnology, and specifically to the use of a neural cell population for treating neurotraumatic diseases, neurodegenerative diseases, neurodevelopmental disorders, or other neurological diseases, disorders, or conditions associated with death and/or dysfunction of forebrain neural cells. The neural cell population comprises forebrain neural progenitor cells (fNPCs) with defined developmental characteristics.

### Background

During early embryogenesis, the forebrain differentiates into the telencephalon and diencephalon. The telencephalon further develops into the cerebral cortex and subcortical structures such as the basal ganglia, olfactory bulb, and hippocampus, while the diencephalon forms the thalamus, hypothalamus, and epithalamus. Owing to the structural and functional complexity of forebrain regions, numerous diseases and disorders are linked to injury or dysfunction of forebrain neural cells. These include
neurotraumatic diseases, such as stroke, post-stroke sequelae, traumatic brain injury (TBI), and post-traumatic neurological deficits; Neurodegenerative diseases, such as Alzheimer's disease (AD), amyotrophic lateral sclerosis (ALS); Neurodevelopmental disorders, such as Epilepsy, autism spectrum disorder, and cerebral palsy.

Stroke is a leading cause of forebrain neural damage and functional impairment worldwide. According to the World Health Organization (WHO), approximately 15 million people suffer strokes annually, with one-third resulting in mortality and another third in permanent severe disability.

In China alone, there are approximately 20 million survivors of post-stroke sequelae, with approximately 2.7 million new cases each year. The aging population exacerbates this burden, highlighting the urgent need for therapies to restore neural function.

Stroke is categorized into hemorrhagic stroke and ischemic stroke, with the latter accounting for approximately 80% of all cases. The brain is highly sensitive to ischemia. Once ischemia occurs, the brain immediately develops functional impairment due to insufficient energy supply. If the condition persists, the impairment will progressively worsen and lead to cerebral infarction.

The primary therapeutic approaches for ischemic stroke involve rapid thrombolysis or endovascular thrombectomy initiated within 3-6 hours post-stroke to restore cerebral blood flow and minimize tissue damage. These interventions include the use of pharmacological agents such as tissue plasminogen activator (t-PA), the first thrombolytic drug approved by the U.S. FDA in 1996 for ischemic stroke management. A critical limitation lies in the narrow time frame (3-6 hours post-onset) for intervention, which often precludes timely patient access to treatment. Another limitation lies in the inability of these therapies to reverse neural damage, resulting in persistent functional deficits and residual complications post-treatment due to ischemic-induced neuronal injury.

In addition, neuroprotective agents are also used in the treatment of stroke, including calcium channel blockers (such as Nimodipine), free radical scavengers (such as Edaravone, Tocopherol, and SOD), neurotrophic factors (such as nerve growth factor), NMDA antagonists, gangliosides, excitatory glycine receptor antagonists, and Butylphthalide. These agents are primarily indicated for acute stroke patients (within ≤14 days post-onset), aiming to mitigate neurological damage, suppress inflammation, and attenuate apoptosis, thereby reducing functional impairment. However, most exhibit suboptimal clinical efficacy in controlled trials, frequently accompanied by adverse effects (e.g., hypotension, hepatotoxicity). Critically, they fail to alleviate chronic post-stroke sequelae such as hemiplegia, owing to an inability to restore disrupted neural circuits or reverse permanent tissue loss.

Cellular therapy represents an emerging therapeutic approach currently under investigation. Clinical trials utilizing mesenchymal cells in acute-phase stroke patients have demonstrated their potential anti-inflammatory effects, promotion of tissue self-repair, and stimulation of vascular regeneration. However, in large-scale controlled clinical trials or studies involving expanded patient cohorts, mesenchymal cells have not yet exhibited statistically significant therapeutic efficacy.

Neurorehabilitation remains the standard therapeutic intervention for functional impairments caused by neurological injuries such as stroke. However, it demonstrates only partial efficacy in promoting functional recovery, with minimal improvements observed during the stable phase of hemiplegia (≥6 months post-stroke).

While existing therapies may mitigate stroke-induced damage or achieve limited functional restoration, they fail to restore functional integrity in damaged neural tissues and ameliorate hemiplegic sequelae.

Most stroke survivors experience chronic disability due to irreversible neural cell loss. Reversing or compensating for such neurodegeneration is clinically imperative. There were no FDA-approved neuroprotective agents can regenerate lost neurons. And no pharmacologic interventions have demonstrated statistically significant functional improvements in patients with stabilized hemiplegia to date.

Research has demonstrated that specific neurogenic niches (the hippocampal dentate gyrus and subventricular zone ) in the adult human brain retain neuroregenerative capacity, exhibiting compensatory neurogenesis following ischemic stroke. However, therapeutic strategies aimed at promoting this kind of endogenous neurogenesis for therapeutic purposes have been very limited in terms of the number of new nerve cells generated and the repair effect, and are unable to overcome the functional deficits caused by brain tissue damage after stroke.

Advancing stem cell technologies have driven research into intracerebral transplantation of tissue-derived neural stem cells (NSCs) in ischemic stroke patients, with the goal of promoting tissue repair and replenishing neuronal loss.

Patent application WO2011137117A1 filed by ReNeuron Ltd. discloses a therapeutic protocol utilizing the immortalized human fetal neural stem cell line CTX0E03 for stroke treatment. Key innovation resides in non-invasive cerebrovascular delivery through intravenous or intra-arterial administration, circumventing the need for craniotomy. The methodology was validated in rodent stroke models. Intervention must be administered within 7 days post-stroke, preferably within a optimal at ≤48 hours post-ischemia.

In the patent application WO2006055685A2 filed by ReNeuron Ltd, a method for treating neurodegenerative diseases by transplanting human neural cells was disclosed. The characteristic of this method is the isolation of neural stem cells from the corresponding tissue region that requires neuronal replenishment, followed by culturing and expanding these cells *in vitro* before transplanting them into the corresponding neural tissue. This process aims to supplement the cells that produce specific neurotransmitters within the neural circuits. However, this application does not target stroke and its associated neural damage, and it specifically requires the use of primary neural stem cells/progenitor cells derived from tissues.

The cells used in the aforementioned method were all derived from neural stem cells obtained from aborted fetal tissues, which are subject to ethical restrictions in clinical commercial applications. Moreover, since the timing of obtaining the so-called"neural stem cells" is relatively late, the cells obtained from the fetal brain or spinal cord tissues at the abortion stage are mainly glial progenitor cells. Consequently, after transplantation into the cranial cavity of animal, the CTX0E03 showed that only 2% differentiated into FOXA3 neurons and about 20% differentiated into astrocytes. There have been no reports of the transplanted cells exhibiting electrophysiological functions or forming synaptic connections with the original animal neurons, indicating that the transplanted cells failed to differentiate into functionally neurons capable of establishing new synaptic circuitry. (EJ Smith, et al., Stem Cell 2012; 30:785-796).

On the other hand, immortalized neural stem cell products (which incorporate oncogenes, fluorescent reporter genes, etc., and are capable of continuous proliferation) can be expanded *in vitro* and produced in batches. However, they pose a certain risk of tumor formation in clinical applications.

Similar to the neural damage caused by stroke, there are many other diseases characterized by the death and/or dysfunction of brain nerve cells, including the neural injury-related diseases mentioned above, neurodegenerative diseases, and neurodevelopmental diseases. It is expected that these diseases can be improved through effective cell replacement therapy for patients. Therefore, there is an urgent need in this field for a cell product that does not rely on fetal tissue as its source and can differentiate into brain nerve cells with the desired functions, thereby providing compensatory cell therapy for neurodegenerative diseases or neural injuries.

### Summary

To address the aforementioned challenges, the inventors developed a forebrain neural progenitor cell (FNPC) population derived through directed differentiation of pluripotent stem cells. These FNPCs were administered to treat a primate (*cynomolgus monkey, Macaca fascicularis*) stroke model with ischemia-induced neural damage, where they were employed to investigate the therapeutic capacity of cells in addressing cerebral neuronal injury and neurodegenerative pathologies.

The inventors demonstrated that the forebrain neural progenitor cells (FNPCs) exhibited prolonged survival post-administration in primate stroke model animals, with a high differentiation ratio into neurons. These cells significantly improved clinical manifestations in diseased animals, providing therapeutic efficacy including enhanced motor function recovery, reduced lesion volume, and increased body weight gain. These findings indicate the potential utility of these FNPCs in treating human neurological disorders, particularly in neural injury repair, neurodegenerative disease management, and related pathological conditions.

The inventors further discovered that the forebrain neural progenitor cells (FNPCs) of the present invention are suitable for administration during the post-acute phase following stroke onset and confer sustained therapeutic improvement. For example, when administered at high doses to cynomolgus monkey (*Macaca fascicularis*) models of permanent middle cerebral artery occlusion (pMCAO), animals receiving FNPC transplantation at 15 days post-infarction demonstrated recovery of horizontal ladder walking speed to healthy baseline levels within 5 months post-treatment. In contrast, saline-administered control animals required approximately twice the duration to complete the task compared to healthy animals at the same timepoint. These results unexpectedly indicate a significant extension of the previously recognized therapeutic window for stroke intervention. The FNPCs or cell-based formulations disclosed herein enable treatment of stroke and associated neural injuries beyond the traditional acute treatment window, including administration during subacute and chronic phases. The present invention was developed based on these findings.

The present invention provides, in a first aspect, a cell population for use in the manufacture of a medicament for treating a subject with nerve injury diseases, neurodegenerative diseases, neurodevelopmental diseases, or other neurological disorders related to death and/or dysfunction of forebrain neural cells, wherein said cell population comprises forebrain neural progenitor cells (FNPCs) possessing defined developmental characteristics. Preferably, the forebrain neural progenitor cells with specific developmental characteristics highly express markers including FOXG1, PAX6, and NESTIN. Preferably, the cell population consists of a specific type of cells, wherein the majority are neural progenitor cells, preferably forebrain neural progenitor cells. Preferably, the cell population is obtained by culturing human pluripotent stem cells (hPSCs) using a specific method.

In a second aspect, the present invention provides a method for treating neurological injury diseases, neurodegenerative diseases, neurodevelopmental diseases, or other neurological disorders associated with the death and/or dysfunction of forebrain neural cells in a subject in need thereof, the method comprising administering to the subject a therapeutically effective amount of a cell population comprising forebrain neural progenitor cells with specific developmental characteristics.

In preferred embodiments of the first and second aspects, the cell population is obtained by culturing human pluripotent stem cells (hPSCs), including embryonic stem cells (ESCs) or induced pluripotent stem cells (iPSCs).

In the preferred embodiments of the first and second aspects, in the cell population, at least about 70% or more, preferably 80% or more, more preferably 90% or more of the cells express one or more markers selected from the group consisting of NESTIN, FOXG1, PAX6, and SOX2. Preferably, in the cell population, at least about 70% or more, preferably at least about 80% or more, more preferably 85% or more, and even more preferably 90% or more of the cells express NESTIN; at least about 70% or more, preferably at least about 80% or more, more preferably 85% or more, and even more preferably 90% or more of the cells express FOXG1; at least about 70% or more, preferably at least about 80% or more, more preferably 85% or more, and even more preferably 90% or more of the cells express PAX6; and at least about 70% or more, preferably at least about 80% or more, more preferably 85% or more, and even more preferably 90% or more of the cells express SOX2.

In the preferred embodiments of the first and second aspects, in the cell population, at least about 70% or more, preferably 80% or more, more preferably 90% or more of the cells express one or more markers selected from the group consisting of: EFNB2, WNT7B, RSPO2, and FEZF2. Preferably, in the cell population, at least about 70% or more, preferably at least about 80% or more, more preferably 85% or more, and even more preferably 90% or more of the cells express EFNB2; at least about 70% or more, preferably at least about 80% or more, more preferably 85% or more, and even more preferably 90% or more of the cells express WNT7B; at least about 70% or more, preferably at least about 80% or more, more preferably 85% or more, and even more preferably 90% or more of the cells express RSPO2; and at least about 70% or more, preferably at least about 80% or more, more preferably 85% or more, and even more preferably 90% or more of the cells express FEZF2.

In the preferred embodiments of the first and second aspects, the neural progenitor cells in the cell population constitute ≥60% of all cell types, preferably ≥70%, more preferably ≥80%. Preferably, the pluripotent stem cells in the cell population constitute ≤3% of all cell types, preferably ≤2%, more preferably ≤1%.

In preferred embodiments of the first and second aspects, the cell population is capable of further differentiating into neuronal cells both *in vitro* and *in vivo.* Preferably, the neuronal cells generated by differentiation of the cell population exhibit neural electrophysiological functionality. Preferably, the neuronal cells generated by differentiation of the cell population express MAP2 and/or TUJ1. Preferably, the neuronal cells account for approximately 80% or more of the total cells in the cell population at 35 days post-differentiation. Preferably, the cell population further generates astrocytes through differentiation. Preferably, the astrocytes generated by differentiation of the cell population express GFAP and/or S100β. Preferably, the astrocytes account for approximately 20% or less of the total cells in the cell population at 35 days post-differentiation."

In specific embodiments of the first and second aspects, the neuropathic injury disorder is stroke or a stroke-associated disease, condition, or symptom, such as sequelae or complications. In these embodiments, the cell population is preferably administered at least 14 days post-stroke onset.

In specific embodiments of the first and second aspects, the neuropathic injury disorder is cerebral palsy or cerebral palsy-associated diseases, conditions, or symptoms, such as sequelae or complications.

In specific embodiments of the first and second aspects, the cell population is administered at a dose ranging from approximately 1×10⁴ to 1×10¹⁰ cells, preferably at a dose ranging from approximately 1×10⁵ to 6×10⁷ cells. For example, when the subject is human, the cell population is administered at a dose ranging from 2×10⁵ to 2×10⁹ cells, preferably at a dose ranging from approximately 2×10⁶ to 1.2×10⁹ cells.

In specific embodiments of the first and second aspects, the cell population is administered to the subject in a single or multiple doses.
In specific embodiments of the first and second aspects, the administration is performed via injection. Preferably, the injection is a local injection.

The advantages of the present invention are at least manifested in the following aspects:
(1) regarding the neurological repair efficacy, treatment with the forebrain neural progenitor cells of this invention demonstrated significantly accelerated recovery in cerebral infarction model animals. Specifically, high-dose cell transplantation resulted in faster weight gain in treated animals compared to controls, and behavioral changes (comprehensive neurological function scores) showing nearly 8-week earlier improvement within 6 weeks, and the achievement of normal healthy monkey-level ladder walking speed in high-dose groups at 7 months post-transplantation. In addition, in cerebral palsy models, high-dose treatment induced up to ^{~}50% recovery of motor/balance functions versus controls. This exceptional efficacy may derive from the forebrain neural progenitor cells of the present invention demonstrating differentiation patterns analogous to normal human brain development, coupled with their ability to form functional neural networks.
(2) Regarding the therapeutic window, the application and methods of the present invention are not confined to the acute phase typically associated with current treatments and medications for related diseases (e.g., 1-3 days post-stroke). They can even be administered more than 14 days after a stroke, thereby offering an option for patients who have missed the acute phase treatment or continue to suffer from neurological impairments despite treatment. This addresses a gap in the current therapeutic landscape.
(3) Regarding the source of the cell , compared to methods that utilize neural stem cells derived from fetal tissue, the neural progenitor cells of the present invention are sourced from pluripotent stem cells such as iPSCs or ESCs, obtained through specific induction and differentiation protocols. This approach offers the advantage of enabling the stable and consistent production of cell products in multiple batches, with a single batch yielding enough cells for hundreds of individuals. It also allows for stringent quality control and circumvents the ethical concerns associated with the use of fetal tissue.
(4) Compared to neural stem cells, neural progenitor cells are more committed progenitors capable of more rapid, efficient, and high-fidelity differentiation into region-specific functional neural cells, thereby reducing risks of aberrant post-transplant proliferation. Conversely, relative to terminally differentiated neurons, these progenitor cells exhibit enhanced cryostability and post-engraftment survival rates, rendering them clinically superior as therapeutic agents.

In summary, the method of the present invention enables the treatment of stroke indications during the non-acute phase, potentially addressing the unmet clinical need in therapeutic approaches for post-stroke hemiplegia sequelae. Simultaneously, by utilizing forebrain neural progenitor cells with specific developmental characteristics to achieve cell and tissue replacement *in vivo,* it can more effectively ameliorate functional impairments caused by brain injury.

### Brief Description of Figures

FIG.1 demonstrates the immunofluorescence identification images at day 7 and 14 of induced differentiation, illustrating the expression of DAPI, MAP2, and FOXG1 through fluorescence of different colors.
FIG.2 demonstrates immunofluorescent characterization of cortical neuron-specific markers at day 35 post-differentiation induction.
FIG.3 demonstrates immunofluorescent characterization of excitatory and inhibitory neuronal markers at day 35 post-differentiation induction.
FIG.4 demonstrates immunofluorescent characterization of presynaptic membrane protein and postsynaptic membrane protein markers at day 35 post-differentiation induction.
FIG.5 demonstrates immunofluorescent characterization of astrocytic and neuronal markers at day 35 post-differentiation induction.
FIG.6 demonstrates immunofluorescent characterization of cortical neuronal markers at day 70 post-differentiation induction.
FIG.7 demonstrates immunofluorescent characterization of excitatory and inhibitory neuronal markers at day 70 post-differentiation induction.
FIG.8 demonstrates immunofluorescent characterization of astrocytic and neuronal markers at day 70 post-differentiation induction.
FIG.9 demonstrates immunofluorescent characterization of oligodendrocyte markers at day 70 post-differentiation induction.
FIG.10 demonstrates electrophysiological profiling of induced cortical neurons at day 70 post-differentiation induction.
FIG.11 illustrates the experimental design for hNPC01 therapy in cerebral infarct model animals, including treatment timelines and assessment protocols.
FIG.12 demonstrates body weight growth rates of cerebral infarct model animals pre- and post-hNPC01 treatment (0-28 weeks), with X-axis indicating days post-treatment.
FIG.13 demonstrates dynamic changes in neurological behavioral scores on the affected side of permanent cerebral infarct model animals treated with different hNPC01 doses (high, medium, low) from 0 to 28 weeks, displayed as percentages relative to the score on day 2 post-treatment (set as baseline (0 point)). The X-axis's day 0 corresponds to day 2 post-treatment (i.e., day 17 post-stroke).
FIG.14 shows individual data points for neurological function behavioral scores of the affected side in cerebral infarction model animals treated with hNPC01 (from the day before treatment to 4 weeks post-treatment).
FIG.15 demonstrates the mean time (s) for a single ladder-walking test in cerebral infarction model animals treated with hNPC01 (5-7 months post-treatment).
FIG.16 presents brain magnetic resonance imaging (MRI) scans of cerebral infarction model animals treated with hNPC01 at Day 183 post-treatment, with each subpanel showing different transverse sections of the same animal.
FIG.17 shows the brain lesion volume (%) in cerebral infarction model animals treated with hNPC01.
FIG.18 presents representative bright-field images of brain tissue and brain sections from cerebral infarction model animals treated with hNPC01 (7 months post-treatment).
FIG.19 demonstrates immunofluorescence staining of the brain in cerebral infarction model animals treated with hNPC01 (7 months post-treatment).
FIG.20 shows the rotarod test results for cerebral palsy model animals treated with hNPC01.
FIGs 21A and 21B display the balance beam test outcomes in cerebral palsy model animals after hNPC01 treatment over a specified period. FIG.21A shows the latency (time taken to cross the beam), and FIG.21B indicates the number of slips.
FIG.22 presents the TTC (2,3,5-triphenyltetrazolium chloride) staining results of cerebral palsy model animals treated with hNPC01.
FIG.23 illustrates the comparative analysis of cellular composition between hNPC01 and fetal forebrain samples.

### Detailed Description

Unless specifically defined herein, all technical and scientific terms used in this invention shall be construed in accordance with their ordinary meaning as understood by a person of ordinary skill in the art to which this invention pertains.

The term 'or' as used herein is synonymous with 'and/or' unless expressly stated otherwise.

In the context of this invention, unless otherwise specified, the terms 'comprising,' 'including,' and 'containing' shall be interpreted to mean encompassing the listed elements-e.g., a component, feature, step, or group thereof-without excluding any other elements, such as additional components, properties, or steps. As used herein, the term 'comprising' or any variation thereof may be replaced with 'containing,' 'including,' 'having,' or synonymous variations. In certain embodiments, 'comprising' further includes the circumstance of 'consisting essentially of' or 'consisting of.'"

### Cell Therapy Products

One characteristic of the present invention lies in the use of specific cells, namely forebrain neural progenitor cells (FNPCs) or cell populations predominantly comprising said cells, as cellular therapeutic agents. Unless otherwise specified, the therapeutic cells used in the invention are of human origin.

In preferred embodiments, the forebrain neural progenitor cells may be derived from human pluripotent stem cells (PSCs), such as embryonic stem cells (ESCs) or induced pluripotent stem cells (iPSCs), through directed differentiation induction, wherein said PSCs serve as the starting material for differentiation.

In preferred embodiments, the cell population of the invention is obtained via a defined differentiation protocol. To facilitate understanding of this protocol, the cell types involved at each differentiation stage are defined below.

The term "cell population" refers to a collection of multiple cells, which may comprise one or more cell types.

The term "pluripotent stem cells" or its abbreviation "PSCs" refers to a class of cells in animals and humans possessing the capacity for self-renewal and differentiation into multiple lineages.

The term "embryonic stem cells" or "ESCs" denotes pluripotent stem cells derived from the inner cell mass of a blastocyst formed by a fertilized egg, capable of differentiating into all germ layer tissues of the human body and self-renewing. Notably, this term excludes fetal stem cells.

The term "induced pluripotent stem cells" or "iPSCs" refers to self-renewing pluripotent stem cells reprogrammed from human somatic cells, capable of differentiating into all germ layer tissues.

The term "neural cells" encompasses all cells constituting the human nervous system.

The term "neural progenitor cells (NPCs)", as used herein, refers to cells exhibiting limited self-renewal capacity that can differentiate into region-specific brain cells or neural lineage cells (e.g., neurons and glial cells).

The term "forebrain neural progenitor cells (FNPCs)" as used herein refers to neural progenitor cells capable of differentiating into neural cells of a specific brain region, namely the forebrain (predominantly including the cerebral cortex). The distinction between FNPCs (or region-specific NPCs) and general NPCs lies in the expression of region-specific markers. FNPCs specifically express markers, whereas general NPCs lack expression of such region-specific markers. For example, forebrain NPCs typically express markers such as FOXG1and PAX6, midbrain NPCs typically express markers such as FOXA2 and LMX1A. Furthermore, upon further differentiation, FNPCs predominantly generate six-layered cortical neurons and glial cells of the cerebral cortex, midbrain NPCs primarily differentiate into midbrain dopaminergic neurons.

The term "embryoid bodies" or "EBs," as used herein, refers to cell aggregates formed from ESCs and iPSCs through three-dimensional culture.

The terms "neural aggregates derived from rosette-forming neural stem cells," "rosette-forming neural aggregates," or "RONA" are used interchangeably in this invention to refer to a specific type of aggregate derived from ESCs or iPSCs, which consists of neural stem cells that spontaneously organize into highly compact three-dimensional neural aggregates.

The term "Neurosphere"as used herein refers to a spherical aggregate formed by neural cells through suspension culture. A neurosphere constitutes a heterogeneous population comprising multiple neural cell types, including neural stem cells (NSCs), neural progenitor cells (NPCs), and differentiated neural cells.

"ECM" stands for extracellular matrix, which is primarily composed of proteins such as collagen, elastin, and/or laminin.

The term "cell preparation" refers to a formulation wherein cells constitute the primary active component, and may include a defined quantity of pharmaceutically acceptable excipients. For example, a cell preparation may be a cell injection solution, such as a sodium chloride injection solution containing said cells. In certain contexts, "cell preparation" and "cell population" may be used interchangeably. For instance, a cell population produced via a specific manufacturing process may be directly administered as a therapeutic agent. Such a cell population may be derived from: A cell culture obtained through the culturing methods disclosed in the present invention; or A derived cellular product generated by further processing said culture (e.g., purification, expansion, or differentiation).

In a preferred embodiment of the invention, at least 70% of the cells in the population are forebrain neural progenitor cells, preferably at least 75%, more preferably at least 80%, even more preferably at least 85%, for example 90% or more, 91% or more, 92% or more, 93% or more, 94% or more, 95% or more, 96% or more, 97% or more, 98% or more, or 99% or more.

The identity of the forebrain neural progenitor cells of the present invention can be determined by one or more of the following methods:1) Morphological observation, 2) Detection of cell-specific biomarkers; and 3) Verification of their ability to differentiate in vitro into specific neural cell types (e.g., cortical neurons) exhibiting both lineage-specific biomarkers and electrophysiological activity.

For example, the forebrain neural progenitor cells of the invention may exhibit classic neural progenitor morphology, including uniform morphology, bright spindle-shaped morphology and a subset of cells organizing into rosette-like arrangements.

For example, the cellular identity of the cell population of the present invention may be jointly determined through neural stem cell markers and forebrain neural progenitor cell markers. Said markers may be selected from the group consisting of: NESTIN, SOX2, DCX, FOXG1, and PAX6. In preferred embodiments, the majority of cells in the cell population or cell preparation of the invention express FOXG1, PAX6, and NESTIN. The majority of cells in the cell population or cell preparation of the invention express FOXG1, PAX6, NESTIN, and SOX2. In a preferred embodiment, in the cell population of the present invention, at least about 70% or more, preferably about 80% or more, more preferably about 85% or more, and even more preferably about 90% or more of the cells express NESTIN; at least about 70% or more, preferably about 80% or more, more preferably about 85% or more, and even more preferably about 90% or more of the cells express FOXG1; at least about 70% or more, preferably about 80% or more, more preferably about 85% or more, and even more preferably about 90% or more of the cells express PAX6; and/or at least about 70% or more, preferably about 80% or more, more preferably about 85% or more, and even more preferably about 90% or more of the cells express SOX2. In a more preferred embodiment, at least about 70% or more, preferably about 80% or more of the cells in the cell population of the present invention simultaneously express NESTIN, FOXG1, and PAX6. In an even more preferred embodiment, at least 70% or more, preferably 80% or more of the cells in the cell population of the present invention simultaneously express NESTIN, FOXG1, PAX6, and SOX2. The expression of these markers can be determined by conventional methods in the field, such as flow cytometry.

The abbreviation "FOXG1"or "Foxg1" are used herein, refers to Forkhead box protein G1, it is one of the earliest expressed transcription factors during human brain development, inducing the development of the telencephalon into several key structures, including the cerebral cortex.

The abbreviation "PAX6" as used herein, refers to Paired Box 6, a determinant of human neuroectoderm cell fate and an important transcription factor for the development of the forebrain.

The abbreviation "NESTIN," as used herein, represents "Nestin protein," a type VI intermediate filament protein that serves as a marker for neural stem cells.

The abbreviation "SOX2," as used herein, represents "Sex determining region Y-box 2," a crucial transcription factor for maintaining pluripotency and self-renewal, and also a marker for neural stem cells.

The abbreviation "DCX," as used herein, represents "Doublecortin," a marker for neural stem cells and neural progenitor cells.

In a preferred embodiment, the cell population or cell preparation of the present invention expresses one or more, preferably all, of the characteristic markers selected from the group consisting of: EFNB2, WNT7B, RSPO2, and FEZF2. "Characteristic markers" refer to the co-expression of these markers, which represents the gene expression signature that distinguishes the cell population of the present invention from products of different developmental stages, cell types, and/or compositions. In a preferred embodiment, the cell population or cell preparation of the present invention simultaneously expresses EFNB2, WNT7B, RSPO2, and FEZF2. In specific embodiments, the expression of these markers can be determined by conventional methods in the field, such as single-cell transcriptome sequencing (e.g., scRNA-seq).

The abbreviation "EFNB2", as used herein, represents Ephrin B2, which is a transmembrane receptor on the cell surface and an important regulator of angiogenesis.

The abbreviation "WNT7B" , as used herein, represents "Wnt Family Member 7B."

The abbreviation "RSPO2" as used herein, represents "R-spondin 2."

The abbreviation "FEZF2" as used herein, represents "FEZ Family Zinc Finger ."

In some embodiments, the cell population of the present invention, in addition to primarily comprising neural progenitor cells, particularly forebrain neural progenitor cells, also includes specific types of diverse cells, comprising one or more, preferably all, selected from the group consisting of: immature neurons, GABAergic neurons, glutamatergic neurons, ependymal cells, and pericytes. Preferably, the proportion of said cells does not exceed 30% of the total cell count, more preferably does not exceed 20%, even more preferably does not exceed 10%, and still more preferably does not exceed 5%.

In some embodiments, the cell population of the present invention is derived from human pluripotent stem cells, wherein the proportion of pluripotent stem cells in said population does not exceed 3% of the total cell population, preferably does not exceed 2%, more preferably does not exceed 1%.

In some embodiments, the forebrain neural progenitor cells or cell populations comprising said progenitors of the present invention, exhibit differentiation capability into neuronal cells both *in vitro* and *in vivo,* preferably forming neuron-dominant cell populations. When the differentiation occurs in vitro or in vivo, particularly in vivo, the differentiation is spontaneous, meaning it does not require the addition of additional reagents such as inducers. Furthermore, the forebrain neural precursor cells or the cell population containing said forebrain neural precursor cells used as a cell therapeutic agent in the present invention exhibit a differentiation trajectory and outcome that resemble the developmental process of a normal human brain.

For example, the cell population of the present invention, when used as a cell therapeutic agent, is capable of expressing neuron-specific markers after differentiation. For instance, the neuronal cells generated by the differentiation of said cell population express MAP2 and/or TUJ1. Both MAP2 and TUJ1 are markers of mature neurons. Preferably, as determined by the expression of MAP2 and/or TUJ1, the cell population of the present invention comprises more than half of the total cells as neuronal cells at around 35 days of differentiation. For example, at around 35 days of differentiation, the neuronal cells in the cell population account for more than about 50%, 60%, 70%, or 80% of the total cells.

For example, the cell population used as a cell-based therapeutic agent in this invention contains cells that express markers of different cortical-specific neurons after differentiation. For instance, among the MAP2+ neuronal cells generated through differentiation in the cell population of this invention, there are neuronal cells that express the marker BRN2 of cortical layers II-IV. For instance, among the MAP2⁺ neuronal cells generated through differentiation in the cell population of this invention, there are neuronal cells that express the marker CTIP of cortical layers V-VI. These cortical-specific neuronal cells may appear around 35 days after differentiation. This means that the therapeutic cells or cell populations of this invention are capable of differentiating into various types of neurons that resemble the normal structure of the human brain.

Critically, the neuronal cells derived from the differentiation of the therapeutic cell population of the present invention demonstrate functional neurophysiological activity. For example, during electrophysiological assessments conducted at approximately day 25 post-differentiation, said differentiated neuronal cells exhibit one or more of the following properties, active electrode count ≥5; weighted mean firing frequency ≥0.5 Hz; burst frequency ≥0.05 Hz.

Preferably, around Day 35 after differentiation in vitro, the cell population can differentiate into cells that co-express synapsin and PSD95. The co-expression of synapsin and PSD95 indicates that synaptic structures have been successfully established between neurons, forming the basis for information exchange between neurons

Preferably, when differentiated in vitro to approximately day 35, the cell population comprises VGLUT+ neurons (glutamatergic excitatory markers) and VGAT+ neurons (GABAergic inhibitory markers), with VGLUT+ neuronal quantities substantially exceeding VGAT+ populations. This excitatory/inhibitory balance recapitulates the natural cortical dominance of glutamatergic neurons, wherein VGLUT+ cells constitute >80% of human neocortical neurons.

During brain development, immature neuronal cells initially predominate, but upon maturation, neurons become postmitotic and cease to proliferate. On the other hand, astrocytes are continuously generated, leading to a gradual increase in their proportion. Therefore, it is preferable that the cell population of the present invention, when used as a cell therapeutic agent, also generates astrocytes through differentiation. Preferably, the astrocytes produced by the differentiation of said cell population express GFAP and/or S100β. Both GFAP and S100βare astrocyte-specific markers. Preferably, the astrocytes generated by the differentiation of said cell population account for approximately 20% or less of the cells produced at around 35 days of differentiation.

In specific embodiments, the cell population used as a cellular therapeutic agent in the present invention is capable of differentiating in vitro into≥80% neuronal cells with high expression of MAP2+ and/or TUJ1+ markers, <20% astrocytes expressing GFAP+/S100β+ markers, while exhibiting functional neuroelectrophysiological properties by approximately day 35 of differentiation. Upon in vivo differentiation, the inventive cell population comprising forebrain neural progenitor cells differentiate into predominantly neuronal populations within approximately 30-90 days post-transplantation.

In preferred embodiments, the cell population of the present invention, when administered as a cellular therapeutic agent to the site of neural injury and adjacent tissue regions in a subject, is capable of establishing reconstructed neural circuitry at the administration site post-differentiation.

In a preferred embodiment, the cell population of the present invention is capable of secreting cytokines and microRNA after transplantation. These cytokines exert neuroprotective and angiogenesis-promoting effects, which contribute to achieving nerve repair outcomes.

### Cell Preparation

The following provides exemplary preparation methods for the forebrain neural progenitor cells of the present invention and the cell populations containing them. Those skilled in the art should understand that this does not imply that the specific steps of said methods cannot be adjusted or modified, nor does it preclude starting from any intermediate step, provided that cell products with comparable properties and compositions can be obtained. The present invention is not limited to completing every step of the methods listed below.

In specific embodiments, the forebrain neural progenitor cells of the present invention or a cell population comprising said forebrain neural progenitor cells are generated from human pluripotent stem cells, such as ESCs or iPSCs, via directed differentiation, wherein the method comprises the following steps:
(1) Culture and expand ESCs or iPSCs in a human pluripotent stem cell culture medium (hPSC medium);
(2) Dissociate ESCs or iPSCs from step (1) and culture in suspension using EB medium to form embryoid bodies (Embryoid bodies, EBs)
(3) Culture the EBs obtained in step (2) under adherent conditions using neural induction medium (RONA) to form rosette-like neural aggregates (Rosette Neural Aggregates, RONAs) ;
(4) Culture the RONAs (rosette-like neural aggregates) formed in step (3) under suspension conditions to generate neurospheres containing forebrain neural progenitor cells (FNPCs);
(5) Optionally, further culture the neurospheres formed in step (4) using NPC medium to passage and expand them; and
(6) Optionally harvest the FNPCs (forebrain neural progenitor cells) formed in step (5).

Optionally, maintain and expand the ESCs or iPSCs to 80-90% confluency prior to step (1), preferably cultured on laminin-coated culture surfaces. The culture surface may be culture plates.

In step (1) described above, ESCs or iPSCs may be dissociated into single cells or cell aggregates e.g., via digestive enzymes or mechanical dissociation, and subsequently seeded into hPSC medium to induce embryoid body formation. The ESCs or iPSCs may be derived from commercial sources or prepared via any established methods. A person skilled in the art will understand that the ESCs refer to those derived from the inner cell mass (ICM) of blastocysts 5-7 days post-fertilization without in vivo development.

In Step (2), more than one type of the EB culture medium of this invention can be used, for example, two, three, or more types.

In Step (3), more than one type of the RONA of this invention can be used, for example, two, three, or more types.

The culture medium used in Step (4) can be the same as that used in the preceding or subsequent steps. Step (4) can be performed using RONA or NPC culture medium. In one embodiment, the culture medium used in Step (4) is the same as that used in Step (3), specifically RONA.

Preferably, the culture in the above-mentioned Steps (3) and (5) is adherent culture, for example, it can be performed on a culture surface coated with extracellular matrix (ECM). In Step (5), neurospheres can be dispersed using digestive enzymes or by mechanical means.

In the above-mentioned preparation method, the EB culture medium, RONA, and NPC culture medium can all consist of a basal medium and additives. Each culture medium can independently be of clinical grade, preferably of cGMP grade or CTS^{™} grade.

The following is a detailed description of the exemplary culture media used in each step.

### A) hPSC Medium

The hPSC medium can be selected from NutriStem^{®} hPSC XF Medium, Essential 8^{™} Medium, StemFit^{®} Basic03 Medium, StemMACS^{™} iPS-Brew Medium, Stem-Partner^{®} ACF Medium, TeSR^{™}-AOF Medium, TeSR2^{™} Medium, with NutriStem^{®} hPSC XF Medium being preferred.

Optionally, the hPSC medium can be supplemented with a ROCK inhibitor. In a preferred embodiment, the ROCK inhibitor is Y-27632. Preferably, the ROCK inhibitor e.g., Y-27632 is added at a concentration of approximately 10 µM.

### B) EB (Embryoid Body) Medium

The EB medium incorporates factors promoting differentiation toward neuroectodermal lineages (e.g., neurons), which is critical for generating cell populations enriched in forebrain neural progenitors.

The EB medium may comprise:
(i) A basal medium selected from a) to c):
   a) KnockOut^{™} DMEM/F12 Medium alone,
   b) a combination of DMEM/F12 Medium and Neurobasal^{™} Medium,
   c) a combination of KnockOut^{™} DMEM/F12 Medium and Neurobasal^{™} Medium; and
(ii) Supplements containing d) or e), or consisting of d) or e),
   d) N-2 Supplement and GlutaMAX^{™}-I Supplement,
   e) N-2 Supplement, GlutaMAX^{™}-I Supplement, and B-27^{™} Supplement (minus vitamin A).

Optionally, the EB medium comprises (iii) inhibitors. The inhibitors comprise, or consist of, a BMP inhibitor, an AMPK inhibitor, and an ALK inhibitor. Preferably, the EB medium comprises, or consists of, one or more selected from the group consisting of Noggin, SB431542, LDN-193189, DMH-1, and Dorsomorphin. More preferably, the EB medium comprises, or consists of, a combination of SB431542 with one or more of Noggin, LDN-193189, DMH-1, and Dorsomorphin, such as SB431542 combined with one or two of Noggin, LDN-193189, DMH-1, and Dorsomorphin.

In specific embodiments, the EB medium comprises:
(i) a basal medium, which is a combination of KnockOut^{™} DMEM/F12 medium and Neurobasal^{™} medium;
(ii) additives, comprising a combination of N-2 supplement and GlutaMAX^{™}-I supplement; and
(iii) SB431542, Noggin, and Dorsomorphin.
   ii.Preferably, when two basal media are used, they are combined at a 1:1 volume ratio.

Preferably, the additives are added at a 1x concentration.

For example:
Noggin is added at a concentration of 25-100 ng/mL, preferably 40-60 ng/mL, more preferably approximately 50 ng/mL.
Dorsomorphin is added at a concentration of 0.5-2 µM, preferably 0.75-1.5 µM, more preferably 1 µM.
SB431542 is added at a concentration of 5-15 µM, preferably 7-13 µM, more preferably 8-12 µM, most preferably 10 µM.

### C) RONA (Rostral Neural Antecedent) Medium

The RONA medium contains factors promoting differentiation toward forebrain neural lineages, which is critical for generating cell populations enriched in forebrain neural progenitors.

The RONA medium may comprise:
(i) A basal medium selected from a) to c):
   a) KnockOut^{™} DMEM/F12 Medium alone,
   b) a combination of DMEM/F12 Medium and Neurobasal^{™} Medium,
   c) a combination of KnockOut^{™} DMEM/F12 Medium and Neurobasal^{™} Medium; and
(ii) Supplements containing d) or e), or consisting of d) or e):
   d) N-2 Supplement and GlutaMAX^{™}-I Supplement,
   e) N-2 Supplement, GlutaMAX^{™}-I Supplement, and B-27^{™} Supplement (minus vitamin A).

In specific embodiments, a RONA medium comprising the following components is used:
(i) a basal medium, which is a combination of Knockout^{™} DMEM/F12 medium and Neurobasal^{™} medium; and
(ii) additives, comprising a combination of N-2 supplement and GlutaMAX^{™}-I supplement.

In specific embodiments, a RONA medium comprising the following components is used:
(i) a basal medium, which is a combination of Knockout^{™} DMEM/F12 medium and Neurobasal^{™} medium; and
(ii) additives, comprising a combination of N-2 supplement, B-27^{™} Supplement (XenoFree, minus vitamin A), and GlutaMAX^{™}-I supplement.

In specific embodiments, more than one RONA medium is utilized. For example, during the first phase of RONA differentiation, a first RONA medium comprising the following components is used:
(i) a basal medium, which is a combination of KnockOut^{™} DMEM/F12 medium and Neurobasal^{™} medium; and
(ii) additives, comprising a combination of N-2 supplement and GlutaMAX^{™}-I supplement.

During the second phase of RONA differentiation, a second RONA medium comprising the following components is used:
(i) a basal medium, which is a combination of KnockOut^{™} DMEM/F12 medium and Neurobasal^{™} medium; and
(ii) additives, comprising a combination of N-2 supplement, B-27^{™} Supplement (XenoFree, minus vitamin A), and GlutaMAX^{™}-I supplement.
Preferably:
The first phase and second phase of RONA differentiation each last approximately 5-14 days, more preferably about 7 days.

When combining the two basal media, they are mixed at a 1:1 volume ratio.

The additives are added at a 1× concentration.

### D) NPC Culture Medium

The base medium of the NPC culture medium can be Neurobasal^{™} Medium, and the additives for the NPC culture medium are (a) GlutaMAX^{™}-I Supplement and (b) B-27^{™} Supplement minus vitamin A.

Optionally, the NPC culture medium may further contain brain-derived neurotrophic factor (BDNF), and/or glial cell line-derived neurotrophic factor (GDNF), and/or L-ascorbic acid, and/or N6, O2'-dibutyryladenosine 3',5'-cyclic monophosphate sodium salt (DB-cAMP).

The BDNF may be animal-free recombinant BDNF or GMP-grade recombinant BDNF, and/or the GDNF may be animal-free recombinant GDNF or GMP-grade recombinant GDNF.

In specific embodiments, an NPC medium comprising the following components is used:
(i) a basal medium, which is Neurobasal^{™} medium;
(ii) additives, comprising a combination of B-27^{™} Supplement (XenoFree, minus vitamin A) and GlutaMAX^{™}-I supplement; and
(iii) BDNF, GDNF, L-ascorbic acid, and N6, O2'-dibutyryladenosine 3',5'-cyclic monophosphate sodium salt (DB-cAMP).

Preferably:
When combining two basal media, they are mixed at a 1:1 volume ratio.

The additives are added at a 1× concentration.

For example, BDNF is added at a concentration of approximately 5-50 ng/mL, preferably 10-30 ng/mL, more preferably about 20 ng/mL.

For example, GDNF is added at a concentration of approximately 5-50 ng/mL, preferably 10-30 ng/mL, more preferably about 20 ng/mL.

For example, L-ascorbic acid is added at a concentration of 0.02-2 mM, preferably 0.05-0.5 mM, more preferably about 0.1-0.3 mM, most preferably about 0.2 mM.

For example, DB-cAMP is added at a concentration of 0.1-5 mM, preferably 0.2-2.5 mM, more preferably about 0.3-1 mM, most preferably about 0.5 mM.

In the aforementioned preferred preparation method, the high yield of prosencephalic neural progenitor cell-dominated population is ensured by the application of specific neural ectoderm-inducing factors during embryoid body (EB) cultivation, and the use of specific prosencephalic differentiation-promoting factors during RONA formation process. In fact, the neurospheres formed in this optimized protocol already contain prosencephalic neural progenitor cells expressing characteristic biomarkers. Thus, it should be noted that after neurosphere formation, alternative passage methods may also be employed to maintain and expand forebrain neural progenitor cells and/or promote their differentiation into neurons, and/or facilitate their differentiation into neurons.

### Therapeutic Method

The term "subject" in the present invention refers to an animal receiving administration, preferably a vertebrate, more preferably a mammal such as a rodent (e.g., mouse, rat), and most preferably a primate (e.g., monkey, including cynomolgus monkey, rhesus monkey), specifically a human.

The term "effective amount"or "therapeutically effective amount" refers to the amount of a pharmaceutical composition (e.g., comprising human forebrain neural progenitor cells of the invention, such as a cell preparation or cell population) that, when delivered to a subject in need, is sufficient to produce a desired function. The desired function may include delaying manifestation of a disorder, preventing or delaying progression of the disorder, or ameliorating at least one effect of the disorder.

The term "treatment" refers to amelioration, alleviation, or elimination (i.e., cure) of disease symptoms. In some cases, treatment includes prophylactic treatment.

When used for repairing neural injury or treating neurodegenerative disorders and other neurological diseases, the transplantation timing of the forebrain neural progenitor cells or cell preparation of the present invention may be 14 days or more after the occurrence of neural injury in the subject. For example, when the subject is a stroke patient, the cell population of the present invention may be administered at least 14 days after stroke.

The forebrain neural progenitor cells of the present invention, or a cell population comprising said forebrain neural progenitor cells, may be administered via intracerebral routes (e.g., intraparenchymal injection) or intraventricular routes (e.g., intrathecal injection, ventricular catheter placement, or intraventricular injection). In preferred embodiments, the neural precursor cells of the present invention, or a cell population comprising said neural cells, may be locally injected, such as through local injection into infarcted or damaged brain regions. For example, injections may be performed in the peri-infarct motor cortex and/or basal ganglia regions. Such local injections may be achieved with imaging guidance, for example, under 3D Lab navigation systems.

The administration or transplantation of said cells or cell populations, such as through injection, may be performed using injection devices like syringes. The injection device may include a needle, such as a puncture needle, or any suitable needle known in the art. The needle should be configured to be sufficiently smooth and rigid to penetrate target tissues, and long enough to reach the desired location. The needle length may range from approximately 1-25 cm. Parameters such as needle size and gauge may be selected based on factors including cell quantity, volume and formulation of the cell preparation, and the injection site.

For injection, cells for transplantation may be provided as a suspension. For example, the cells of the invention may be suspended in a buffer with suitable osmotic pressure, such as physiological saline (0.9% sodium chloride solution).

When the total volume of cell suspension is large, such as in cerebral parenchymal injections, multiple injections across different depths and positions may be performed through multiple needle tracts (e.g., 2, 3, 4, 5, or more needle tracts), with each tract containing a portion of the total cell suspension volume.

The forebrain neural progenitor cells or cell populations containing them may be administered through single or multiple injections (e.g., 2, 3, or more times). The frequency of injections may be determined based on improvement efficacy. Intervals between multiple injections may range from one or more days, weeks, months, or longer.

In a preferred embodiment, the forebrain neural progenitor cells of the present invention or cell populations containing said cells are administered via intracranial single-site or multisite injections. For example, within a single cranial burr hole, injections may be performed through 1 to 3 needle tracts with distinct insertion angles. For each needle tract, cells may be injected into 1 to 10 different injection sites at varying depths. This may be achieved by pausing at specific depths during withdrawal of the inserted injection device to administer injections. Alternatively, slow, continuous injection during device withdrawal may deposit cells along a segment of the withdrawal path.

The cell population comprising the forebrain neural progenitor cells of the present invention may have a single-dose administration ranging from approximately 1×10⁴ to 1×10¹⁰ cells, such as 1×10⁴ cells, 1×10⁵ cells, 1×10⁶ cells, 1×10⁷ cells, 1×10⁸ cells, 1×10⁹ cells, or 1×10¹⁰ cells. In preferred embodiments, the single-dose administration may range from approximately 1×10⁵ cells to 1×10⁷ cells, such as 1×10⁵, 2×10⁵, 3×10⁵, 4×10⁵, 5×10⁵, 6×10⁵, 7×10⁵, 8×10⁵, 9×10⁵, 1×10⁶, 2×10⁶, 3×10⁶, 4×10⁶, 5×10⁶, 6×10⁶, 7×10⁶, 8×10⁶, or 9×10⁶ cells. In some embodiments, when the subject is human, the cell population is administered at a dose ranging from 2×10⁵ to 2×10⁹ cells, preferably approximately 2×10⁶ to 1.2×10⁹ cells. In a single dose, the forebrain neural precursor cells of the invention should constitute at least 70% of the total cells, preferably at least 80%, such that at least 70% (preferably at least 80%) of the total cells in the single dose express FOXG1, PAX6, and NESTIN.

### Disease/Therapeutic Efficacy Evaluation Metrics

Following administration of the prosencephalic neural progenitor cells or cell populations containing said cells according to the present invention, therapeutic outcomes may be evaluated through multiple metrics.

For instance, within 1 month to 1 year post-transplantation/administration of the cell populations or cell-based preparations of the invention, improvements may be observed in one or more of the following domains, neurological function and motor function assessments for post-stroke sequelae (e.g., Modified Rankin Scale, Fugl-Meyer Motor Scale [FMMS], or NIH Stroke Scale [NIHSS] scores), language capacity, cognitive performance, specific clinical manifestations.

Preferably, such improvements demonstrate statistically significant progression compared to pre-transplantation/administration baselines. For example, quantitative score enhancements exceeding at least 10% (preferably ≥20%) and/or approaching healthy cohort reference ranges. Resolution of specific symptoms, including but not limited to transition from hypertonic clenched fist posture to regained manual dexterity (e.g., writing, utensil operation), patients with unilateral leg weakness or impaired mobility show improvement to the extent of being able to walk freely and/or run independently, among other physical activities; bedridden patients with cognitive impairment show improvement to the extent of being able to recognize family members and/or engage in communication and similar activities; and/or patients with aphasia regain the ability to speak, communicate, and perform related functions.

For example, eligible patients may fall within the age range of 20-85 years. Additionally, inclusion criteria for cerebral infarction may involve cortical/subcortical ischemic infarctions caused by unilateral middle cerebral artery (MCA) or lenticulostriate artery occlusion, with an ischemic lesion size ≥3 cm, and two preoperative assessments spaced 3 weeks apart with FMMS scores <55, among other qualifying parameters.

### Use

The forebrain neural progenitor cells of the present invention, or a cell population comprising said forebrain neural progenitor cells, may be used either as a standalone therapy or in combination with other pharmaceutical agents for the treatment of neurological injury diseases, neurodegenerative diseases, neurodevelopmental disorders, or other neurological diseases, disorders, or conditions associated with the death and/or dysfunction of forebrain neural cells.

The term "brain injury" as used herein specifically refers to damage to cerebral tissue caused by either internal or external forces. This encompasses both mechanical injuries such as contusions classified under intracranial injuries, and pathophysiological injuries induced by ischemia, hypoxia, or reperfusion. Brain injury may result in diverse cognitive and functional impairments, including deficits in attention, memory retention, or motor coordination.

The term "neurological injury diseases"as used herein specifically refers to brain-related neural injuries. Such neurological injury diseases encompass brain injuries caused by cerebral ischemia, cerebral hemorrhage, or physical trauma, which result in neural damage.

The term "neurodegenerative disease" refers to disorders characterized by progressive functional deterioration and demise of specific neuronal populations during disease progression. In specific embodiments, such neurodegenerative diseases include, but are not limited to, amyotrophic lateral sclerosis (ALS), Huntington's disease (HD), Alzheimer's disease (AD).

The term "neurodevelopmental disorders" as used herein specifically refers to pathological conditions resulting from aberrant development of cerebral tissues and cells, with specific emphasis on anomalies affecting prosencephalic (forebrain) structures and cellular populations.

The term "other neurological disorders associated with prosencephalic neuronal death and/or dysfunction" as used herein specifically designates neurological diseases involving damage to forebrain neurons in terms of quantity and/or function.

The term "cerebral apoplexy/stroke" within the context of this invention shall be interpreted broadly to encompass both ischemic and hemorrhagic subtypes. Ischemic stroke, also referred to as cerebral infarction or cerebral infarct, is caused by impaired blood supply to a localized region of brain tissue, resulting in acute interruption of blood flow. This leads to ischemia (insufficient blood flow), hypoxia (oxygen deprivation), softening, necrotic changes, and tissue death in the affected brain area. Clinically, this manifests as rapid-onset neurological deficits, such as hemiplegia (paralysis on one side of the body) and aphasia (loss of language ability). Intracerebral hemorrhage is also known as cerebral hemorrhage.

The term cerebral palsy (CP) within the context of this invention refers to non-progressive cerebral lesions occurring during the perinatal period (prenatal through neonatal stages). These lesions manifest primarily as motor dysfunction, cognitive developmental delays and epileptic seizures.

In specific embodiments, the forebrain neural progenitor cells of the present invention, or a cell population comprising said forebrain neural progenitor cells, can be used to treat neural injury diseases. Said neural injury diseases can be selected from the group consisting of diseases related to ischemic brain injury, diseases related to hemorrhagic brain injury, and diseases related to brain injury caused by trauma.

It is generally recognized in the field that treatment during the acute phase of neural injury yields significantly better therapeutic outcomes. However, even with timely intervention in the acute phase, patients may still suffer disabilities or permanent impairments due to neural damage. The cell population of the present invention is applicable not only to subjects in the acute phase of neurological injury diseases but also to those beyond the acute phase, such as subjects in the stable phase (e.g., chronic phase).Without being bound by theory, the cell population of the present invention may exert therapeutic effects through cytokine release in acute-phase subjects, while in stable-phase subjects, it may achieve functional recovery via mechanisms such as cell replacement-mediated reconstruction of neural circuits.

In specific embodiments, ischemic cerebral injury refers to ischemic stroke, also termed cerebral infarction. For example, the subject has suffered from or has a history of total anterior circulation infarct, partial anterior circulation infarct, posterior circulation infarct, and lacunar infarct, or the patient has suffered from or has a history of cerebral ischemia caused by large artery atherosclerosis, cardioembolic events, and small artery occlusion. Disorders were associated to ischemic brain injury can be stroke or stroke-related conditions, such as complications or sequelae.

In specific embodiments, the hemorrhagic brain injury is hemorrhagic stroke, which can also be referred to as cerebral hemorrhage. Diseases related to hemorrhagic brain injury can be stroke or stroke-related conditions, such as complications or sequelae.

The stroke-related symptoms may include, but are not limited to hemiplegia, hemibody impairment, limb numbness, facial muscle weakness, numbness, aphasia, slurred speech, and hemianopia.

Brain injuries caused by trauma, such as those resulting from contusions or penetrating wounds, or related symptoms such as complications or sequelae.

In preferred embodiments, the forebrain neural progenitor cells of this invention are particularly suitable for the treatment of ischemic brain injury, especially ischemic stroke or its related conditions, such as complications or sequelae. In such cases, the forebrain neural progenitor cells of this invention are suitable for administration after the acute phase of stroke, for example, more than 14 days after the occurrence of stroke.

In specific embodiments, the forebrain neural progenitor cells of the present invention or cell populations comprising the forebrain neural progenitor cells can be used to treat cerebral palsy. The cell therapy of the present invention can improve motor function and/or cognitive function in subjects with cerebral palsy.

In specific embodiments, the forebrain neural progenitor cells of the present invention or cell populations comprising said forebrain neural progenitor cells are applicable for treating neurodegenerative diseases. Neurodegenerative diseases are disorders characterized by the progressive loss of function in neural cells, particularly neurons. Neurodegenerative diseases suitable for treatment with the cells or cell populations of the invention are those involving the loss of function in forebrain neural cells, particularly forebrain neurons, such as Alzheimer's disease (AD), amyotrophic lateral sclerosis (ALS), and Huntington's disease (HD).

In specific embodiments, the forebrain neural progenitor cells of the present invention or cell populations comprising said forebrain neural progenitor cells are applicable for treating neurodevelopmental diseases. Neurodevelopmental diseases, also termed neurodevelopmental disorders (NDDs), are characterized by impaired development of the nervous system. Neurodevelopmental diseases suitable for treatment with the cells or cell populations of the invention are those involving the loss of function in forebrain neural cells, particularly forebrain neurons, such as epilepsy, autism spectrum disorder (ASD), and cerebral palsy (CP).

The forebrain neural progenitor cells of the present invention, or cell populations comprising said forebrain neural progenitor cells, may be used for research purposes, including clinical and non-clinical research. For example, the research may be studies evaluating animal models of the aforementioned diseases. For example, the research may be studies on the therapeutic mechanisms of forebrain neural progenitor cells in the treatment of these diseases, such as injury repair mechanisms.

### Embodiments

The embodiments of the present invention are described below by way of example. Those skilled in the art should understand that this does not mean the present invention must be implemented through the specific methods provided herein. The scope of the invention shall be governed by the claims.

### Embodiment 1. Preparation of Cells

In this embodiment, a cell preparation predominantly composed of forebrain neural progenitor cells (hNPC01) was prepared from human induced pluripotent stem cells (iPSCs) via directed induction differentiation (iPSC→EB→RONA→neurospheres→forebrain neural progenitor cells (FNPCs)).

It should be noticed that this does not imply the implementation of the present invention is necessarily required to start with iPSCs.

### 1. Culture of iPSCs

The cell suspension was seeded at a density of approximately 2×10⁵ viable cells/well onto an MX521 matrix-coated 6-well cell culture plate containing NutriStem hPSC XF Xeno-free medium (supplemented with 10 µM Y-27632). Cells were cultured in a 37°C, 5% CO₂ incubator until reaching 90% confluence.

### 2. Differentiation culture of embryoid bodies (EBs)

Prepare the EB differentiation medium (EB-M) according to the table below.

| **EB-M** | |
|---|---|
| **Component** | **Amount** |
| Knockout^{™} DMEM/F-12 | 125 mL |
| Neurobasal^{™} Medium | 125 mL |
| N-2 Supplement | 2.5mL |
| GlutaMAX^{™}-I Supplement | 1.25 mL |
| Noggin | 125 µL |
| Dorsomorphin | 25 µL |
| SB431542 | 250 µL |

The procedures for EB differentiation are performed as follows:

### (1) Embryoid Body (EB) Seeding

Remove the old medium and wash cells with DPBS (without Ca²⁺/Mg²⁺). Add 1 mL of Cell Therapy Systems TrypLE^{™} Select Enzyme to each well and incubate until large-scale cell detachment occurs. Neutralize digestion by adding 3 mL of medium per well, then transfer the cell suspension to a centrifuge tube.

Centrifuge at 200 g for 3 minutes and discard the supernatant.

Resuspend the cell pellet in NutriStem hPSC XF Xeno-free medium supplemented with 10 µM Y-27632, and perform cell counting.

Seed the cell suspension at a density of ≈0.3×10⁵ viable cells/mL into a low-attachment 96-well cell culture plate. Culture in a 37°C, 5% CO₂ incubator for 48 hours.

### (2) EB Medium Replacement

On Day 2 of differentiation, move EBs from a low-attachment 96-well plate to a 6-well plate (32 EBs/well). From Days 2-6, replace medium daily with 2 mL fresh EB-M, then incubate at 37°C, 5% CO₂ for a total of 7 days.

### 3. RONA Differentiation Culture

The RONA-M1 differentiation medium for Phase 1 (Day 1-Day 7) is formulated as specified in the table below.

| **RONA-M1** | |
|---|---|
| **Medium Preparation** | **Amount** |
| Knockout^{™} DMEM/F-12 | 125 mL |
| Neurobasal^{™} Medium | 125 mL |
| N-2 Supplement | 2.5 mL |
| GlutaMAX^{™}-I Supplement | 1.25 mL |

The RONA-M2 differentiation medium for Phase 2 (Day 8-Day 14) is formulated as specified in the table below.

| **RONA-M2** | |
|---|---|
| **Medium Preparation** | **Amount** |
| Knockout^{™} DMEM/F-12 | 125 mL |
| Neurobasal^{™} Medium | 125 mL |
| N-2 Supplement | 1.25 mL |
| B-27^{™} Supplement, XenoFree, minus vitamin A | 2.5 mL |
| GlutaMAX^{™}-I Supplement | 1.25 mL |

The RONA differentiation procedure is performed as follows.

Transfer embryoid bodies (EBs) from the central regions of low-attachment 6-well plates to MX521-coated 6-well plates pre-filled with 3 mL RONA-M1 medium per well.

Culture the plates in a 37°C, 5% CO₂ incubator to establish RONA structures.

During the RONA culture phase, RONA-M1 medium is used from Day 1 to Day 7, and RONA-M2 medium is used from Day 8 to Day 17.

### 4. Neurosphere Culture

Prepare the NPC-M basal medium for forebrain neural progenitor cells according to the composition table below.

| NPC-M | | |
|---|---|---|
| **Number** | **Medium Preparation** | **Amount** |
| 1 | Neurobasal Medium(Neurobasal Medium) | 483.5 mL |
| 2 | B-27^{™} Supplement, XenoFree, minus vitamin A(B27-XF) | 10 mL |
| 3 | GlutaMAX^{™}-I Supplement(GlutaMAX) | 5 mL |
| 4 | Stock solution: Contains 20 ng/mL Animal-Free Recombinant Human/Murine/Rat BDNF (PeproTech), 20 ng/mL Animal-Free Recombinant Human GDNF (PeproTech), 0.2 mM L-ascorbic acid (VC; Sigma), and 0.5 mM N6,O2'-dibutyryladenosine 3',5'-cyclic monophosphate sodium salt (DB-cAMP; Sigma). | 1.5 mL |

Neurosphere culture is performed as follows. After completion of the RONA differentiation phase, transfer the central cell clusters from RONA structures to 6-well low-attachment plates containing 2.5 mL NPCM. Culture in a 37°C, 5% CO₂ incubator to establish neurospheres.

FNPC inoculation can be conducted after 12-72 h of neurosphere culture, once neurospheres are enriched with FNPC.

### 5. Expansion Culture of Forebrain Neural Progenitor Cells

Collect neurospheres into 50 mL conical tubes and centrifuge at 300 rpm for 3 min. After removing the supernatant, add 10 mL Accutase to the tubes. Incubate in a 37°C, 5% CO₂ incubator for 15 min for digestion. Quench the digestion by adding old medium, followed by centrifugation at 300 g for 3 min. Discard the supernatant, add 3 mL NPCM, and resuspend to form a single-cell suspension. Perform cell counting. The results showed a total of 5.67 × 10⁸ viable cells with a viability of 98%.

Adjust the cell concentration to 1.52 × 10⁶ viable cells/mL using NPCM. Seed cells into T175 culture flasks coated with 8 µg/mL Matrix MX521. Incubate in a 37°C, 5% CO₂ humidified incubator. Replace the medium the next day (46-50 h post-seeding) and subculture on day 4. Cryopreserve cells after reaching the fourth passage.

### 6. Preparation of Cell Products

Resuspend thawed cell stock in 0.9% sodium chloride injection to achieve a cell concentration of 5.0 × 10⁴ viable cells/µL. Aliquot the suspension into transport vials to obtain the final cell product hNPC01.

Alternatively, cell products may be prepared using FNPC cells in active passaging stages.

### 7. Characterization and Quality Assessment of hNPC01 Cell Product

i.Analyze cell markers (FOXG1, PAX6, NESTIN, and SOX2) via flow cytometry. Results demonstrate 93% positivity for FOXG1, 97% for PAX6, >98% for NESTIN, and 94% for SOX2 in hNPC01. These data confirm that the overwhelming majority of cells in the product express the expected FNPC markers.

### Embodiment 2. In Vitro Differentiation Assay

To evaluate the biological activity of the cell product prepared in Example 1, the product underwent directed differentiation. Expression of multiple markers was assessed via immunofluorescence at distinct time points during differentiation, and neural electrophysiological activity was monitored to characterize the differentiation capacity of the cell product.

### 2.1 Differentiation of hNPC01

hNPC01 was subjected to neuronal differentiation using the NPCM (Neural Progenitor Culture Medium) described in Example 1 as the differentiation medium. Cells were plated at a density of 2.5 × 10⁴ viable cells/well into 24-well plates coated with MX521 (BioLamina), with 500 µL of NPCM per well. Adherent growth of hNPCs was observed the following day. The medium was semi-refreshed with fresh NPCM every 3-7 days (partial medium exchange at 50% volume).

### 2.2 Immunofluorescence Staining Assay

The markers detected by immunofluorescence staining and their results at multiple analysis time points are presented in Table 1 below.

**Table 1. Summary of Cellular Marker Detection Results**

| | Day 7 | Day 14 | Day 35 | Day 70 |
|---|---|---|---|---|
| FOXG 1 | 88.00% | 41.91% | / | / |
| MAP2 | + | + | + | + |
| SYNAPSIN | / | / | + | / |
| PSD95 | / | / | + | / |
| CTIP2 | / | / | 37.55% | 75.89% |
| BRN2 | / | / | 20.18% | 8.49% |
| SATB2 | / | / | 6.90% | 13.15% |
| TBR1 | / | / | 33.36% | 18.43% |
| VGAT | / | / | + | + |
| VGLUT1 | / | / | + | + |
| GABA | / | / | + | + |
| GFAP | / | / | + | + |
| GLUR2 | / | / | / | + |
| OLIG2 | / | / | / | 1.37% |

| | | | | |
|---|---|---|---|---|
| Note: "+" indicates that the corresponding cell marker was tested and showed positive at the specified time point; % values indicate that the cell marker was tested at the time point, with results displayed as the percentage of positive cells; "/" indicates that the cell marker was not tested at the time point, and no corresponding result is available. | | | | |

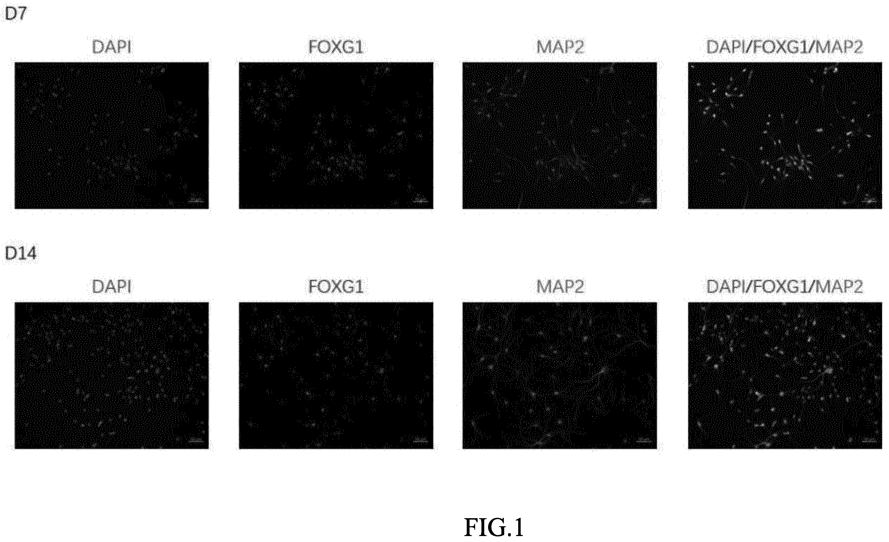

From the results in the table, it can be seen that after 7 days of in vitro induction and differentiation, the neural progenitor cell marker (FOXG1, 88.00%) and the neuronal cell marker (MAP2) (FIG.1) can be detected. This indicates that the majority of cells are still in the neural progenitor cell state.

After 14 days of in vitro induction and differentiation, the neural progenitor cell marker (FOXG1, 41.91%) and the neuronal cell marker (MAP2) (FIG.1) can be detected. The observed decrease in the proportion of cells expressing neural progenitor cell markers indicates that the cells have undergone differentiation.

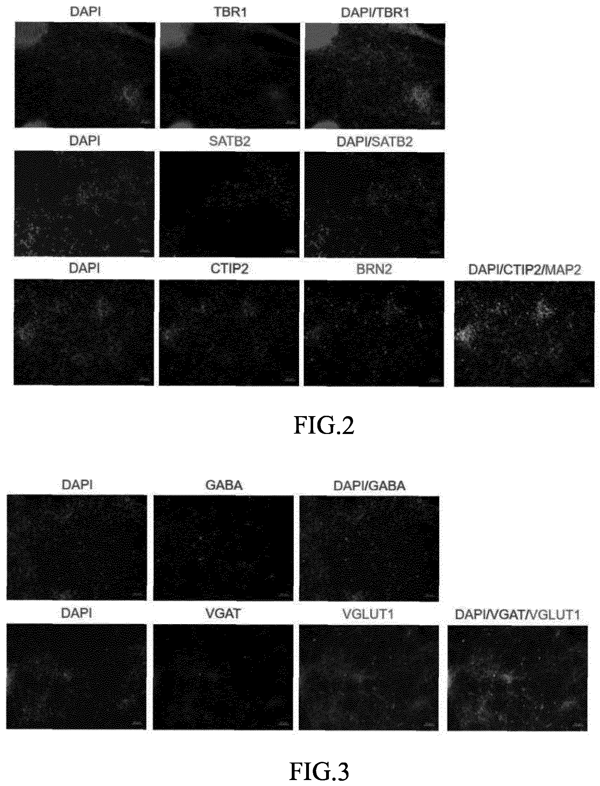

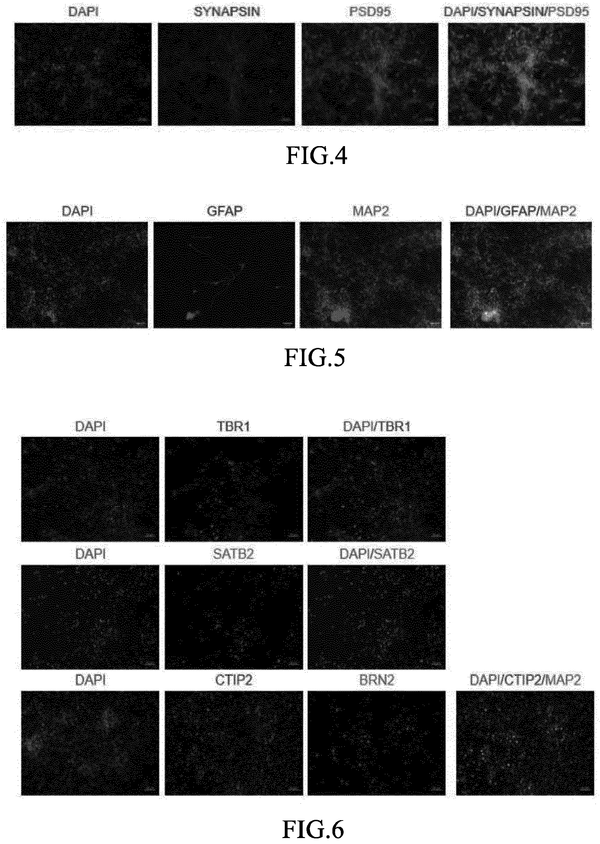

At 35 days of in vitro differentiation, the induced differentiated cells were found to contain >90% neuronal cells and expressed cerebral neuronal markers. Detectable markers of cerebral neuronal cells included layer 6 cortical neuronal markers (CTIP2: 37.55%, BRN2: 20.18%, TBR1: 33.36%, SATB2: 6.90%) (FIG.2), excitatory glutamatergic neuronal marker (VGLUT1, ^{~}70-80%), inhibitory neuron-specific vesicular GABA transporter marker (VGAT, ^{~}20-30%), and inhibitory neuron neurotransmitter GABA marker (GABA, ^{~}10-20%) (FIG.3), presynaptic and postsynaptic membrane protein markers (Synapsin, PSD95, >99%) (FIG.4), astrocyte marker (GFAP, ^{~}2%) and neuronal marker (MAP2, >90%) (FIG.5).

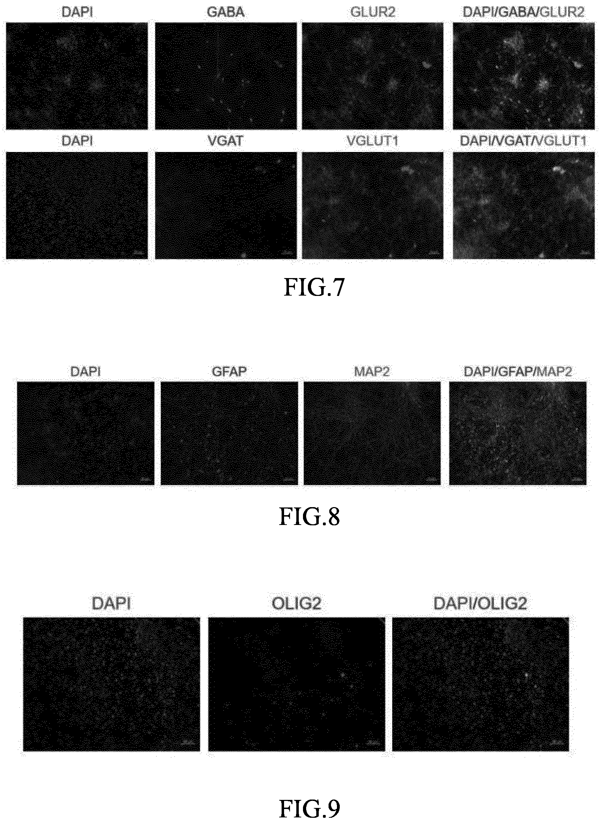

After 70 days of in vitro induction and differentiation, the induced cells were detected to contain >65% neurons, approximately 30% astrocytes, and 1.37% oligodendrocyte progenitor cells. Corresponding brain neuronal cell markers were detected, including markers for layer 6 cortical neurons (CTIP2: 75.89%, BRN2: 8.49%, TBR1: 18.43%, SATB2: 13.15%) (FIG.6); excitatory glutamatergic neuronal markers (VGLUT1, about 70-80%), AMPA receptor GLUR2 subunit markers for excitatory neurons (GLUR2, about 70-80%), inhibitory neuronal markers for the vesicular γ-aminobutyric acid transporter (VGAT, about 20-30%), and inhibitory neuronal markers for the neurotransmitter γ-aminobutyric acid (GABA, about 20-25%) (FIG.7); astrocyte markers (GFAP, about 30%), neuronal markers (MAP2, about 65%) (FIG.8), and oligodendrocyte markers (OLIG2, about 1.37%) (FIG.9).

These results indicate that hNPC01 can differentiate into mature functional human cortical neurons and glial cells, with a cellular composition highly similar to that of the human brain.

### 2.3 Electrophysiological Monitoring

The electrophysiological activity of cells was monitored using multi-electrode arrays (MEA) at days 8, 17, 20, and 25 after hNPC01 differentiation initiation.

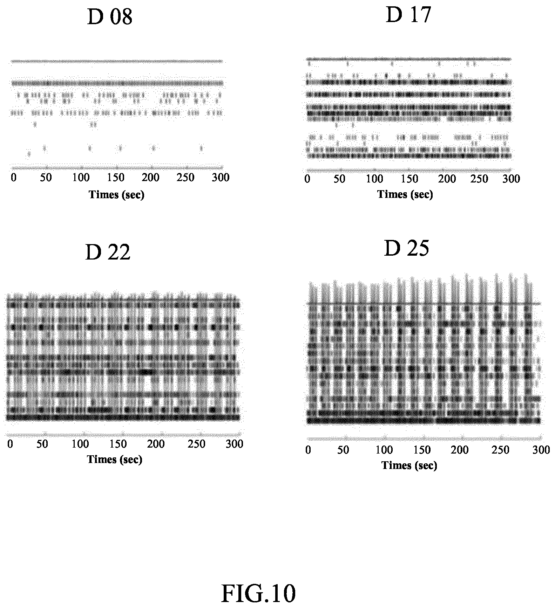

Results in FIG.10 show that spontaneous electrophysiological activity and synchronized firing-characteristics of relative maturity-were detected around 3 weeks of differentiation (day 20). At day 25, the following metrics were recorded,
active electrode is 16, weighted mean firing frequency is 3.2 Hz, and the burst frequency is 0.16 Hz.

These results demonstrate that cortical neurons derived from FNPCs via the induction method exhibit formation of complex neural networks within ^{~}3 weeks, with network complexity increasing progressively over differentiation time.

### Embodiment 3. Cell Therapy in a Cynomolgus Monkey Model of Permanent Ischemic Stroke

In this embodiment, the inventors conducted cell therapy in a primate stroke model using cynomolgus monkeys. The brain volume and functional regions of cynomolgus monkeys closely resemble those of humans, and their post-stroke pathological progression parallels human stroke outcomes.

The permanent ischemic stroke model (pMCAO) was established by surgical ligation of the left M1 segment of the middle cerebral artery in cynomolgus monkeys. MRI and functional assessments confirmed that the model mimics severe human stroke patients, with persistent neurological and motor deficits observed 6-7 months post-infarction. In contrast, rodent reperfusion ischemic models only exhibit functional deficits detectable within 14 days to 1 month. This model addresses the issue of rapid spontaneous recovery in rodents, thus enabling robust simulation of long-term functional deficits in human stroke patients and evaluation of post-acute therapeutic efficacy.

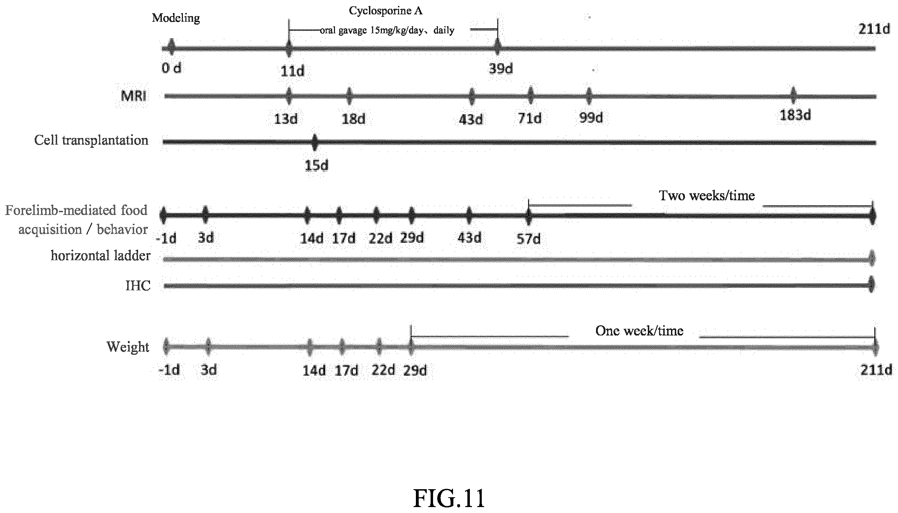

For experimental details, see FIG.11.

### 3.1 Establishment of a Permanent Ischemic Stroke Model

Experimental animals: 24 cynomolgus monkeys, aged 4-8 years, tested negative for Mycobacterium tuberculosis (TB), Simian retrovirus (SRV), Simian T-cell leukemia virus (STLV), and Simian immunodeficiency virus (SIV).

Grouping: Randomly divided into 4 groups with 6 monkeys in each group, with an equal number of males and females. The groups were the model control group, low-dose hNPC01 group, medium-dose hNPC01 group, and high-dose hNPC01 group. In the following text, the model control group is sometimes referred to as the control group, and the latter three groups are collectively referred to as the experimental groups.

Model establishment: On day 0, the left middle cerebral artery (M1 segment) of the cynomolgus monkeys in both the control and experimental groups was permanently ligated with surgical sutures to establish a permanent ischemic stroke model in cynomolgus monkeys.

### 3.2 Cell Injection (Transplantation)

On day 13 (Day 13) after establishing the ischemic stroke model, MRI brain images were acquired from each group to determine infarct locations for guiding injection targeting.

Starting 4 days prior to cell injection (transplantation), Cyclosporine A was orally administered at 15 mg/kg/day for immunosuppression in all groups, continued for 28 days.

On day 15 (Day 15) post-stroke model induction, cynomolgus monkeys received a single intracerebral injection of hNPC01 neural progenitor cells into the peri-infarct motor cortex and basal ganglia regions. The injections utilized an intracranial multi-point approach (3 needle tracks, 3 injection sites per track at varying depths). Dose allocations were model control group with 0 (0.9% sodium chloride injection), hNPC01 low-dose group with 4.5×10⁵ cells/animal, hNPC01 medium-dose group with 9.0×10⁵ cells/animal and hNPC01 high-dose group with 18×10⁵ cells/animal.

### 3.3 Pharmacodynamic Evaluation Protocol

After cell injection, cynomolgus monkeys in each group were continuously observed for 28 weeks for pharmacodynamic and toxicological evaluations. Specific observation/measurement methods and parameters are summarized in Table 2.

**(1) Table 2. Behavioral Observation Criteria and Biochemical Assay Metrics**

| **Method** | **Items** | **Evaluation Content** |
|---|---|---|
| Physiological Monitoring I | Number of animal deaths, baseline animal indicators, body weight, etc. | Mortality rate, overall health condition |
| Behavioral Observation | Cage-side behavior evaluation, feeding behavior of the right upper limb, ladder traction walking | Neurological function and specific motor functions |
| MRI | T1/T2 MRI data of brain tissue metabolic signals | Changes in brain injury and infarct volume |
| Immunohistochemical staining | Following euthanasia at the experimental endpoint, brain sections were prepared and subjected to analysis | Survival, Tissue Distribution, and Differentiation of Transplanted Cells |

### 3.3.1 Physiological Monitoring

All model animals underwent daily clinical observation following middle cerebral artery occlusion surgery. Throughout the trial, vital signs (body temperature, blood pressure) remained normal, animals displayed stable emotional states with no abnormal behaviors, and no deaths occurred.

Body weight measurements were performed 1 day pre-modeling, 3 days post-modeling, 1 day pre-transplantation, and 2 days (D2), 7 days (D7), 14 days (D14), 28 days (D28) post-transplantation, followed by weekly measurements thereafter.

The body weight growth rate of experimental animals (stratified by gender) after cell therapy was calculated as: Body weight growth rate (%) = (Current weight - Pre-modeling weight) / Pre-modeling weight × 100%

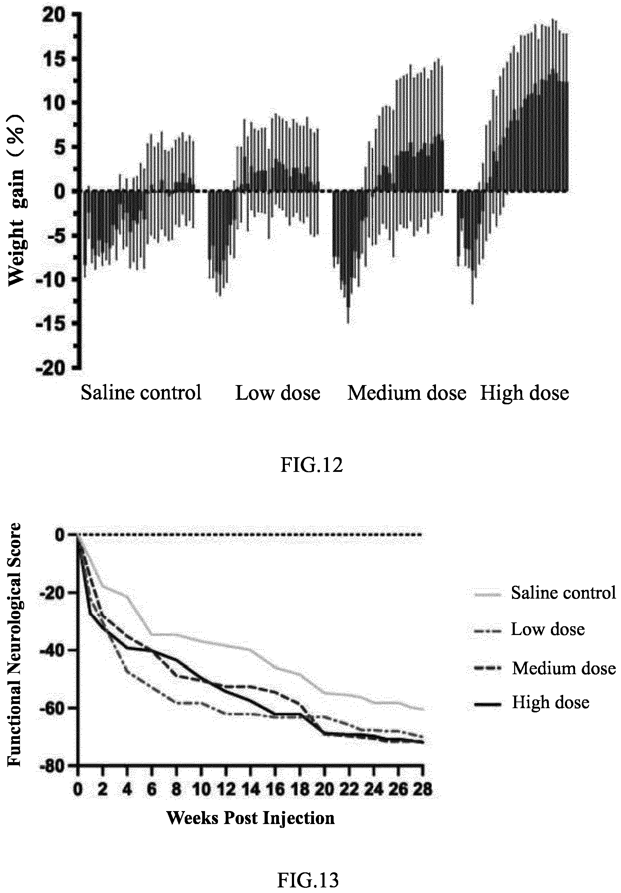

As shown in FIG.12, both medium-dose and high-dose cell transplantation groups exhibited higher weight gain rates than the vehicle control group, with the high-dose group surpassing the medium-dose group. Two-way ANOVA analysis revealed statistically significant differences (p < 0.001) between the high-dose group versus vehicle control, low-dose, and medium-dose groups, indicating faster recovery of appetite and feeding capacity in high-dose group animals post-modeling.

### 3.3.2 Behavioral Observations

Cage-side ethological assessments and forelimb food retrieval skill tests were performed at 1 day pre-modeling, 3 days post-modeling, 1 day pre-transplantation, and 2 days (D2), 7 days (D7), 14 days (D14) post-transplantation, followed by biweekly assessments; weekly ladder rung walking tests were conducted after 176 days post-transplantation.

### 3.3.2.1 Cage-Side Behavior Assessment (Neurological Function Scoring)

Prior to surgical modeling, cage-side ethological evaluations were performed on behaviorally trained and grouped cynomolgus monkeys. The scoring system assessed neurological function based on consciousness level, sensory system (contralateral to ischemic side), motor system, and skeletal muscle coordination. A 0-100 scoring scale was applied, with lower scores indicating closer-to-normal neurological function and higher scores reflecting more severe neurological impairment.

Results demonstrated normal performance in consciousness, sensory system, motor system, and skeletal muscle coordination across all groups pre-modeling. All animals successfully completed caregiver-guided commanded tasks, confirming successful behavioral adaptation training.

At 3 days and 14 days post-modeling via cerebral ischemic ligation (corresponding to 12 days before and 1 day before cell transplantation), cage-side ethological scores showed significant declines. By day 14 post-modeling, marked behavioral deficits in consciousness, sensory system, motor system, and skeletal muscle coordination were observed, with cerebral injury severity meeting predefined success criteria, confirming effective model establishment.

Post-treatment, to exclude behavioral confounds induced by the cell transplantation procedure itself, statistical analyses of cage-side behavioral changes across groups within 28 weeks post-transplantation were performed using Day 3 post-transplantation (D17) as the baseline (time zero). Cage-side evaluations were conducted at: 2 days (D17), 7 days (D22), 14 days (D29) post-transplantation, followed by biweekly assessments. Results are shown in FIGs 13-14.

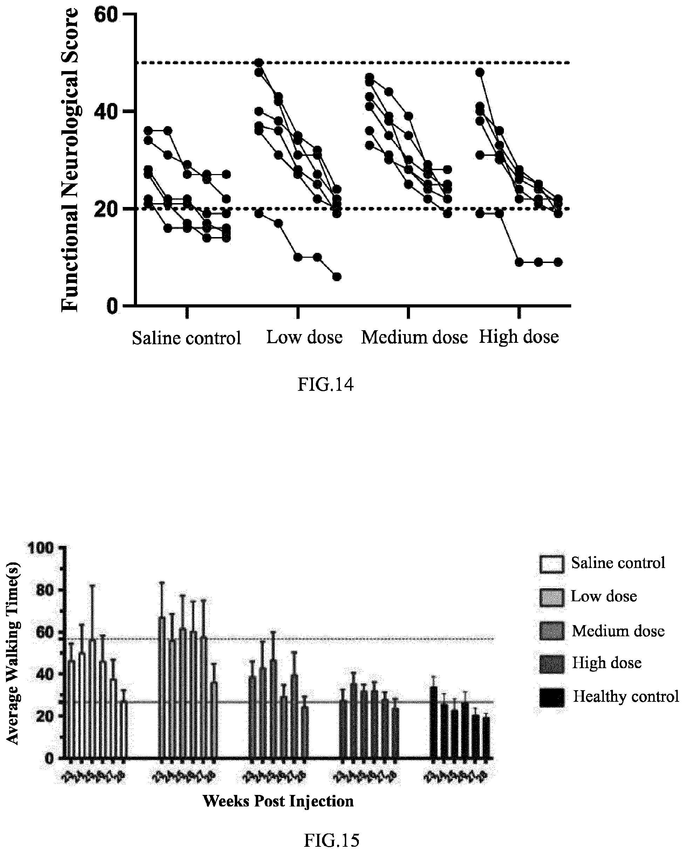

Statistical analysis revealed that low-, medium-, and high-dose cell therapy groups exhibited significantly accelerated behavioral recovery compared to the vehicle control group (p < 0.0001 by two-way ANOVA, FIG.13). Additionally, nearly all treated animals (excluding two with milder modeling injuries) demonstrated 30-50% higher baseline ipsilateral behavioral scores (pre-treatment) versus vehicle controls, indicating more severe initial neurological deficits. Nevertheless, these animals displayed faster recovery rates (FIG.14).

### 3.3.2.2 Ladder Rung Walking Test

To directly investigate functional recovery post-cell therapy and intergroup differences, the ladder rung walking test was added starting from 23 weeks post-treatment (D176).

Animals were guided to traverse a narrow-spaced ladder (rung spacing: 10 cm) for three consecutive rounds. Single-round traversal time and forelimb usage were recorded and statistically analyzed. This test assessed recovery of motor behavior, body coordination, sensory system, and muscle tone.

Additionally, normal non-modeled cynomolgus monkeys (n = 6) were included as a normal animal group for comparative analysis with control and experimental groups. Results are shown in FIG.15.

FIG.15 displays the mean single-round traversal time measured between months 5-7 (weeks 23-28). Two-way ANOVA revealed significant intergroup differences (p < 0.001). By month 5, the high-dose group recovered to levels comparable to the normal non-modeled group, whereas the vehicle control group required nearly double the traversal time.

At 5 months post-treatment with the hNPC01 cell formulation (non-acute phase injection), motor function and coordination of the cerebral ischemia-modeled animals in both forelimbs and hindlimbs recovered to healthy animal levels, achieving this outcome approximately 2 months earlier compared to control groups.

### 3.3.3 Animal Brain Tissue Magnetic Resonance Imaging (MRI)

MRI scans were performed on all groups at 13 days post-modeling (2 days prior to transplantation) to analyze brain injury severity and assess whether the model was successfully established.

Additionally, brain MRI images were collected at 3 days (D18), 28 days (D43), 56 days (D71), 84 days (D99), and 168 days (D183) post-cell transplantation to evaluate changes in brain lesion volume and therapeutic efficacy.

Postoperative (2-week) evaluation of modeled animals demonstrated ischemic stroke symptoms in cage-side ethology, feeding ability, and MRI imaging, confirming successful model establishment.

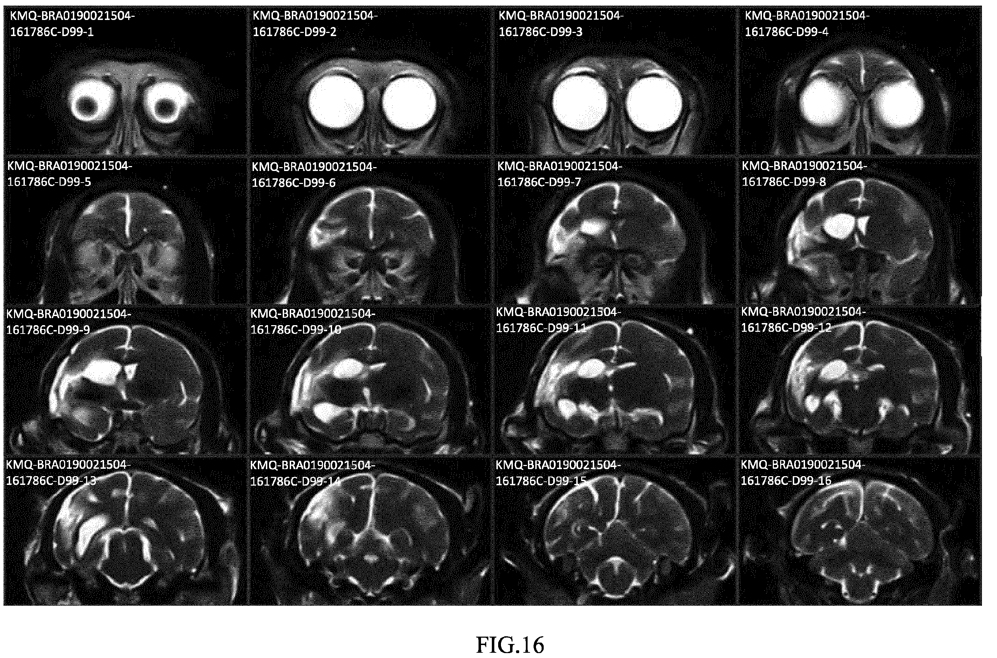

Typical MRI images of animal brains at the experimental endpoint (post-transplantation) are shown in FIG.16.

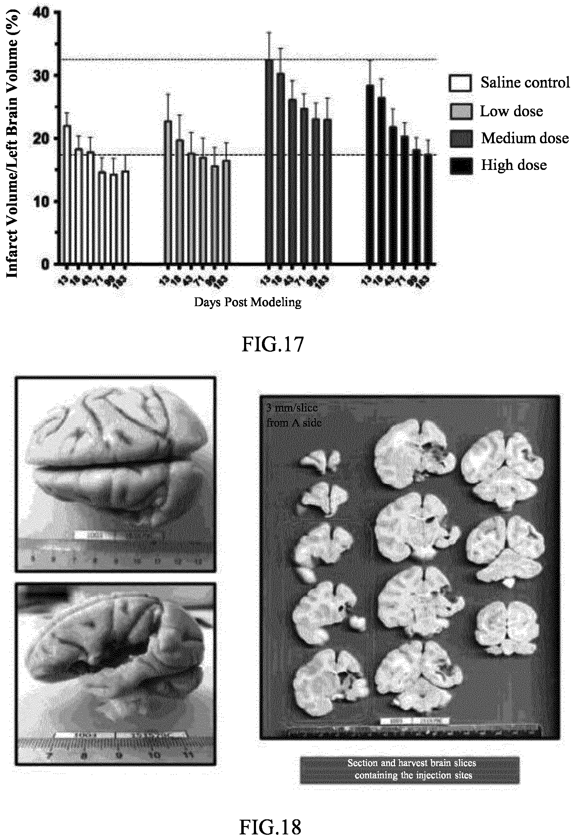

Using 3D Slicer, statistical analysis of the proportion of brain lesion volume was performed based on MRI images, with results displayed in FIG.17.

As shown in FIG.17, maximum brain injury severity in all groups occurred prior to cell transplantation. Compared to baseline (pre-treatment, D14), the high-dose hNPC01 group exhibited the greatest reduction in brain lesion volume within 4 weeks post-treatment, followed by the medium-dose and low-dose groups. These results suggest that hNPC01 cell therapy improves post-acute-phase ischemic stroke brain injury/infarction and promotes tissue repair, with higher doses demonstrating superior efficacy.

### 3.4 Immunohistochemical Staining

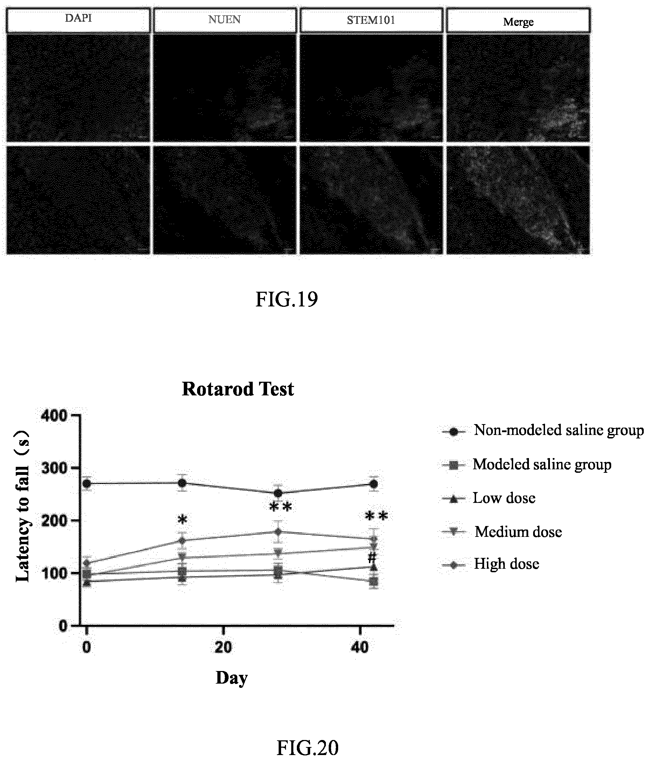

At the experimental endpoint, all remaining animals were euthanized, and brain tissues were collected (FIG.18). Brain tissues from a subset of animals underwent pathological examination: the infarcted-side brain tissues were fixed, coronally thick-sectioned, and scanned. H&E staining was performed on lesion-area brain tissues to observe inflammatory cell infiltration. Immunofluorescence staining results from the medium-dose group are shown in FIG.19, where anti-human nuclear marker STEM101 and mature neuron marker NEUN were used.

As shown in FIG.19, >80% of human-derived cells (identified by STEM101 staining) in brain slices from hNPC01-treated animals expressed the mature neuronal marker NEUN. This indicates that human neural progenitor cells predominantly differentiated into mature neurons 7 months post-transplantation.

In summary, all the results suggest that after the cell preparation hNPC01, which contains forebrain neural progenitor cells (FNPCs) induced from iPSCs, is transplanted into model animals, the experimental group that receives cell therapy shows significant improvements in neurological and locomotor functions compared with the control group following treatment. Additionally, recovery of body weight and brain tissue injury is observed. The transplanted FNPCs were able to differentiate into neurons. Furthermore, in some experiments, the therapeutic effect of FNPCs demonstrates a dose-dependent manner, indicating that a specific higher dose may be more preferable.

### Embodiment 4. Cell Therapy in a Rat Model of Hypoxic-Ischemic Cerebral Palsy

In this embodiment, the inventors tested the therapeutic efficacy of the cells of the present invention in an animal model of hypoxic-ischemic cerebral palsy to demonstrate the effectiveness of the cell product in treating diverse types of brain injury.

### 4.1 Modeling and Administration Protocol

A classical Rice-Vannucci model was employed. On postnatal day 7 (P7), SPF-grade Sprague-Dawley (SD) rats underwent permanent ligation of the unilateral common carotid artery. After a 1-hour postoperative recovery, hypoxia was induced by exposing the rats to a gas mixture of 8% oxygen (balanced with 92% nitrogen) for 2.5 hours. Successful modeling was confirmed by monitoring motor asymmetry in the limbs and unilateral eye opening in the pups. On day 7 post-modeling, 10 µL of hNPC01 cells at varying densities was stereotaxically injected into the lateral ventricle using a brain stereotaxic apparatus. Saline was administered in the same manner to both non-modeled and modeled rats as controls. Experimental groups are detailed in Table 3.

**Table 3. Treatment Protocols for Experimental and Control Groups**

| Number | Group | Number | Injection | Cell |
|---|---|---|---|---|
| | | of animals | | concentration |
| G1 | Non-modeled saline group | 10 | Saline | NA |
| G2 | Modeled saline group | 10 | Saline | NA |
| G3 | Low dose group | 10 | hNPC01 injection | 2*10E3 |
| G4 | Medium dose group | 10 | hNPC01 injection | 2*10E4 |
| G5 | High dose group | 10 | hNPC01 injection | 1*10E5 |

### 4.2 Pharmacodynamic Evaluation

Pharmacodynamic evaluation was conducted during a continuous 10-week observation period following cell injection in all rat groups. Specific experimental protocols included rotarod test, balance beam test and 2,3,5-Triphenyltetrazolium Chloride (TTC) staining assay.

### 4.2.1 Rotarod Test

Rotarod tests were conducted before administration and at 2, 4, and 6 weeks post-administration to assess the effect of hNPC01 on motor function in rats.

Animals were transferred from housing to the laboratory and acclimatized for 60 minutes prior to testing. Rats were placed on a rotating rod with a diameter of 90 mm. After a 2-minute adaptation period on the stationary rod, the rotation speed was set to an initial rate of 4 rpm and increased to 40 rpm over 300 seconds. The duration of each rat's sustained movement on the rotating rod was recorded. After completion of testing, animals were returned to their housing, and the apparatus was cleaned with 70% ethanol.

It should be noted that the temperature, humidity and lighting intensity of consistent laboratory conditions were maintained throughout all tests.

The rotarod test results as shown in FIG.20, the high-dose group demonstrated significantly longer duration on the rotating rod compared to the modeling saline group at 2 weeks, 4 weeks, and 6 weeks post-administration (*, P< 0.05 ; **, P<0.01 ; **, P<0.01). The high-dose group exhibited stable performance with no deteriorating trends observed. The medium-dose group showed a statistically significant increase in movement duration at 6 weeks compared to the modeling saline group (#, P < 0.05).

No significant differences were detected between the low-dose group and the modeling saline group at any timepoint.

### 4.2.2 Balance Beam Test

Balance beam tests were performed before administration and at 2, 4, and 6 weeks post-administration to evaluate the effect of hNPC01 on motor function in rats.

During the training phase, animals were transferred from the housing room to the laboratory and acclimated for 60 minutes. Training was conducted using a 25mm square column. The animal was placed at the starting end of the crossbeam, and a stopwatch was used to record the time taken to traverse the central 100cm distance of the crossbeam into the dark box, defined as the latent period. Measurements included the latent period on the diameter crossbeam and the number of slips by the left/right hind paws. After testing, animals were returned to the housing room, and the apparatus was cleaned with 70% ethanol. It should be noted that the temperature, humidity and lighting intensity of consistent laboratory conditions were maintained throughout all tests.

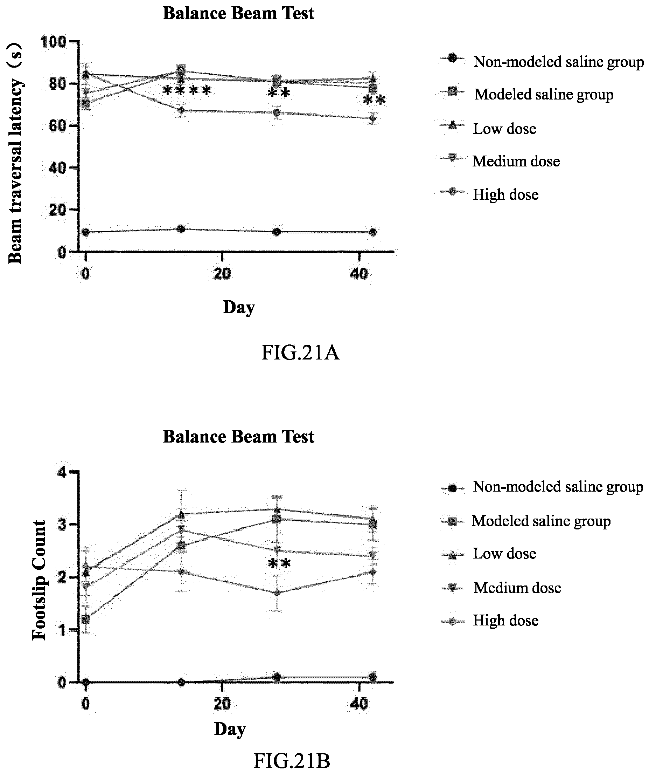

The balance beam test results are shown in FIGs 21A and 21B. The high-dose treatment group demonstrated significantly shorter traversal times compared to the model saline group at 2, 4, and 6 weeks post-administration (****, P < 0.0001; **, P < 0.01), with stable performance and no observed deterioration trends. No significant differences or worsening trends were observed between the medium-dose group, low-dose group, and the model saline group at any time points.

### 4.2.3 TTC Staining

TTC staining was performed at the end of the experimental observation. Dehydrogenases in viable cells of normal brain tissue can reduce TTC to insoluble red-stable triphenyltetrazolium formazan (TTF). If brain tissue cells are dead or have reduced viability, they will exhibit weak or no staining. Therefore, the vitality of brain tissue can be assessed based on the staining location and intensity.

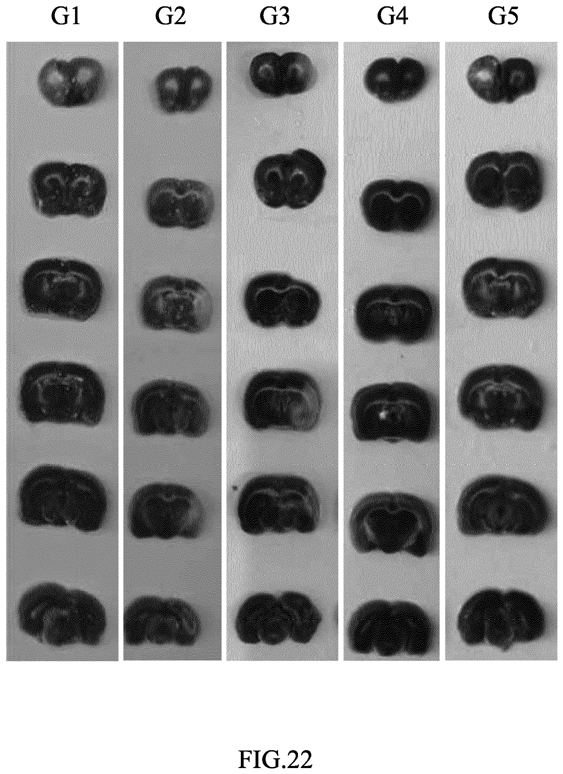

The TTC staining results are shown in FIG.22. No significant residual damage was observed in the non-modeled saline group (G1), medium-dose treatment group (G4), or high-dose treatment group (G5). Significant cerebral tissue damage was observed in both the low-dose treatment group and the modeled saline group.

### Embodiment 5. Cellular Composition Study of hNPC01

This embodiment describes the study of the cellular composition of hNPC01 using single-cell RNA sequencing (scRNA-seq) technology. Additionally, hNPC01 was compared with neural progenitor cells (NPCs) from the developing fetal forebrain, with the fetal forebrain NPC data obtained from a published article (Zhong, S., et al., A single-cell RNA-seq survey of the developmental landscape of the human prefrontal cortex. Nature 2018, 555 (7697), 524-528). Through data comparison, it was found that the transcriptomic data and molecular markers of hNPC01 are consistent with those of naturally occurring human forebrain neural progenitor cells.

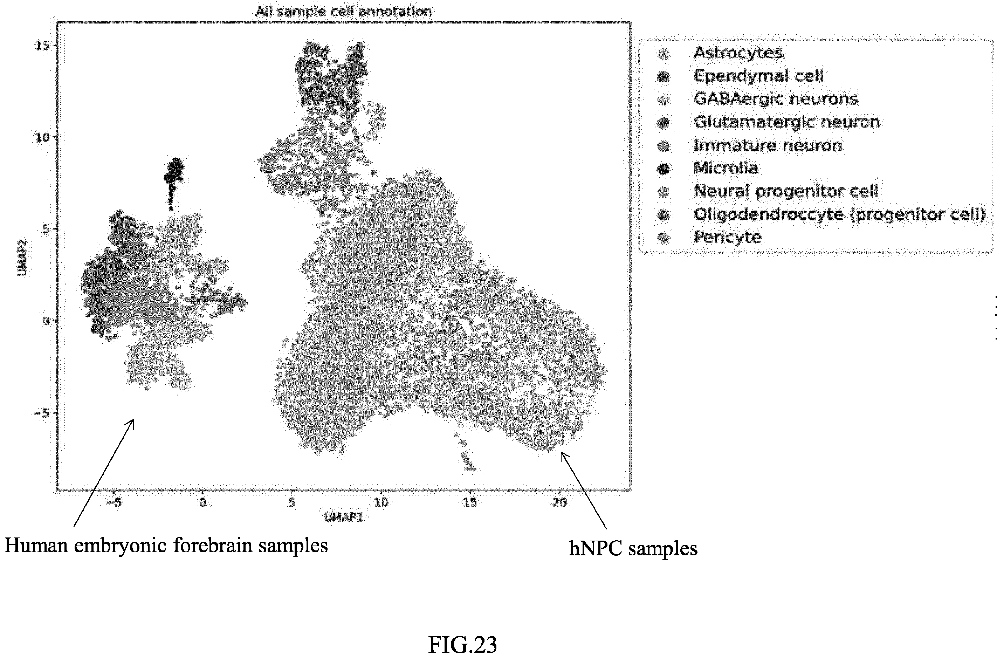

Based on the results of scRNA-seq, the cell types in the hNPC01 samples were annotated, and the phenotypes of the cells in the samples were confirmed by the profiles of gene expression, with the results shown in FIG.23. As shown in FIG.23, the main cell type in the hNPC01 sample is neural progenitor cells, which are phenotypically consistent with the main cell type in the fetal forebrain sample, and their proportion in the hNPC01 sample is ≥70%. In addition, the hNPC01 sample also includes immature neurons, GABAergic neurons, glutamatergic neurons, and a small number (≤10%) of ependymal cells and pericytes that are widely present in the brain. The neural progenitor cells in hNPC01 can further differentiate into cortical neurons with correct subcellular composition, mature electrophysiological functions, and the ability to survive long-term *in vivo* and *in vitro* (including various excitatory and inhibitory neurons, astrocytes, and oligodendrocyte progenitor cells). Moreover, these transplanted neural progenitor cells can secrete a variety of neuroprotective and angiogenic cytokines and microRNAs, which contribute to enhancing the endogenous repair processes.

Further analysis of the sequencing data from the fetal samples compared with the data from the hNPC01 samples revealed a high degree of consistency in the expression profiles of neural progenitor and neuronal markers between the two types of samples.

The comparison of the scRNA-seq data showed that the forebrain NPC cell product hNPC01 derived from iPSC induction and differentiation in this invention has the same types of neural progenitor - related characteristic genes as the naturally occurring fetal neural progenitor cells. However, the expression levels of pluripotent stem cell marker genes are lower compared to those in fetal cells, indicating fewer or less proliferative cells with strong division capabilities. This result demonstrates that there is no residual iPSC - phenotypic cells in the cell product of this invention, nor are there cells with mesodermal or endodermal phenotypes. This suggests that the cell product of this invention has a higher purity of forebrain neural progenitor cells and is therefore safer.

Further comparative analysis of the differences with single - cell sequencing data from human embryonic brain development, as well as single - cell sequencing data from organs derived from human mesodermal and endodermal development, revealed that the cell product of this invention specifically over - expresses molecular markers of forebrain neural progenitor cells. These include EFNB2, WNT7B, RSPO2, and FEZF2, among others. This result also supports the conclusion that the cell product of this invention has a high purity of forebrain neural progenitor cells.

### Embodiment 6. In Vivo Neural Circuit Reconstruction Study

In addition to assessing whether transplanted cells survive and differentiate into the desired types, the ability of differentiated cells to form functional neural circuits is a critical indicator of transplantation efficacy. This study demonstrates that the hNPC01 cells of the present invention, when transplanted into the ischemic lesion area of immunodeficient mice, not only further differentiate into mature neural cells *in vivo* but also integrate into the host's existing central nervous system, forming functional synaptic connections to achieve neural circuit reconstruction. This process is crucial for functional recovery following brain injury.

After transplanting 2×10⁵ hNPCs into the peri-infarct region of the somatosensory cortex in each rat, the recombinant viral vector scAAV2/1-hSyn-Cre was injected into the ventral thalamus of the animals. This serotype of AAV exhibits retrograde synaptic labeling capability, inducing Cre recombinase expression in downstream neurons. Concurrently, another recombinant viral vector, AAV2/8-EF1α-DIO-mCherry, was injected into the somatosensory cortex of the same animal. Neurons infected with this virus express the mCherry fluorescent reporter gene upon encountering Cre recombinase transmitted from upstream neurons, resulting in inversion of the LoxP sequence. Red mCherry signals were observed in the AAV2/8-EF1α-DIO-mCherry injection sites, indicating direct synaptic connections between neurons in this region and the ventral thalamus. During the experiment, red mCherry signals were first detected in the normal somatosensory cortex, confirming the validity of this strategy.

Furthermore, red mCherry signals were successfully observed in GFP-positive progeny neurons derived from transplanted human forebrain NPCs. These signals colocalized with anti-human nuclear antibody (HNA) staining, further confirming that the progeny neurons of transplanted hNPCs established direct synaptic connections with the host's ventral thalamus, demonstrating successful neural circuit reconstruction.

## Claims

1. Use of a cell population comprising human forebrain neural progenitor cells for treating a neurological disorder in a subject, said disorder selected from the group consisting of neurological injury diseases, neurodegenerative diseases, neurodevelopmental disorders, and other neurological diseases, conditions, or disorders associated with death and/or dysfunction of forebrain neural cells; or in the manufacture of a medicament for treating said neurological disorder.

2. The use according to claim 1, wherein in said cell population, at least about 70% of cells express NESTIN, at least about 70% of cells express FOXG1, at least about 70% of cells express PAX6, and/or at least about 70% of cells express SOX2.

3. The use according to claim 2, wherein in said cell population, at least about 70% of cells express NESTIN, at least about 70% of cells express FOXG1, at least about 70% of cells express PAX6.

4. The use according to claim 3, wherein in said cell population, at least about 70% of cells express NESTIN, at least about 70% of cells express FOXG1, at least about 70% of cells express PAX6, and at least about 70% of cells express SOX2.

5. The use according to claim 4, wherein in said cell population, at least about 80% of cells express NESTIN, at least about 80% of cells express FOXG1, at least about 80% of cells express PAX6, and at least about 80% of cells express SOX2.

6. The use according to any one of claims 1-5, wherein said cell population expresses one or more genes selected from the group consisting of EFNB2, WNT7B, RSPO2, and FEZF2.

7. The use according to claim 6, wherein said cell population co-expresses EFNB2, WNT7B, RSPO2, and FEZF2.

8. The use according to any one of claims 1 to 7, wherein said cell population, neural progenitor cells, preferably forebrain neural progenitor cells, constitute at least 60% of all cell types, more preferably at least 70% of all cell types, still more preferably at least 80% of all cell types.

9. The use according to any one of claims 1 to 8, wherein said cell population further comprises one or more cell types selected from the group consisting of, immature neurons, GABAergic neurons, and glutamatergic neurons.

10. The use according to any one of claims 1-9, wherein the cell population further comprises ependymal cells and/or pericytes.

11. The use according to claim 10, wherein in said cell population, the total proportion of immature neurons, GABAergic neurons, glutamatergic neurons, ependymal cells, and pericytes does not exceed 30% of the total cell count, preferably does not exceed 20%, more preferably does not exceed 10%, and even more preferably does not exceed 5%.

12. The use according to any one of claims 1-11, wherein the forebrain neural progenitor cells are obtained from human pluripotent stem cells (hPSCs) *via* an induced differentiation method.

13. The use according to claim 12, wherein said inducing differentiation method comprises the following steps:
**(1)** culturing and expanding ESCs or iPSCs in a human pluripotent stem cell (hPSC) medium;
**(2)** digesting the ESCs or iPSCs from step (1) and subjecting them to suspension culture in an EB medium to form embryoid bodies (EBs);
**(3)** adherently culturing the EBs obtained from step (2) using a neural induction medium (RONA) to form rosette neural aggregates (RONAs);
**(4)** picking neural ectodermal cells from the RONAs formed in step (3) and subjecting them to suspension culture to form neurospheres.

14. The use according to claim 13, wherein said inducing differentiation method further comprises after step (4):
**(5)** culturing the neurospheres formed in step (4) in an NPC medium for passaging and expansion.

15. The use according to claim 14, wherein said inducing differentiation method further comprises after step (5):
**(6)** harvesting the FNPCs formed in step (5).

16. The use according to any one of claims 13 to 15, wherein said hPSC medium is NutriStem^{®} hPSC XF medium, Essential 8 medium, StemFit^{®} Basic03 medium, StemMACS^{™} iPS-Brew medium, Stem-Partner^{®} ACF medium, TeSR^{™}-AOF medium, or TeSR2 medium.

17. The use according to any one of claims 13 to 16, wherein said hPSC medium is supplemented with a ROCK inhibitor.

18. The use according to claim 17, wherein said ROCK inhibitor is Y-27632.

19. The use according to claim 18, wherein said Y-27632 is added at a concentration of about 10 µM.

20. The use according to any one of claims 13 to 19, wherein said EB medium comprises:
(i) a basal medium selected from the group consisting of:
a) KnockOut^{™} DMEM/F12 medium alone;
b) a combination of DMEM/F12 medium and Neurobasal^{™} medium;
c) a combination of KnockOut^{™} DMEM/F12 medium and Neurobasal^{™} medium; and
(ii) an additive comprising or consisting of:
d) N-2 supplement and GlutaMAX^{™}-I supplement;
e) N-2 supplement, GlutaMAX^{™}-I supplement, and B-27^{™} Supplement minus vitamin A.

21. The use according to claim 20, wherein said EB medium further comprises an inhibitor composition comprising a BMP inhibitor, an AMPK inhibitor, and an ALK inhibitor, or consisting of a BMP inhibitor, an AMPK inhibitor, and an ALK inhibitor.

22. The use according to claim 21, wherein said EB medium further comprises an inhibitor composition comprising one or more selected from the group consisting of: Noggin, SB431542, LDN-193189, DMH-1, and Dorsomorphin, or consisting of one or more selected from the group consisting of: Noggin, SB431542, LDN-193189, DMH-1, and Dorsomorphin.

23. The use according to claim 22, wherein said inhibitor composition comprises a combination of SB431542 with one or more selected from the group consisting of: Noggin, LDN-193189, DMH-1, and Dorsomorphin, or consists of said combination.

24. The use according to claim 23, wherein said inhibitor composition comprises a combination of SB431542 with one or two selected from the group consisting of: Noggin, LDN-193189, DMH-1, and Dorsomorphin, or consists of said combination.

25. The use according to any one of claims 13 to 24, wherein said EB medium comprises:
(i) a basal medium being a combination of KnockOut^{™} DMEM/F12 medium and Neurobasal^{™} medium;
(ii) an additive comprising a combination of N-2 supplement and GlutaMAX^{™}-I supplement; and
(iii) SB431542, Noggin, and Dorsomorphin.

26. The use according to any one of claims 13 to 25, wherein said step (3) employs a RONA medium comprising:
(i) a basal medium being a combination of KnockOut^{™} DMEM/F12 medium and Neurobasal^{™} medium; and
(ii) an additive comprising a combination of N-2 supplement and GlutaMAX^{™}-I supplement.

27. The use according to any one of claims 13 to 26, wherein said step (3) employs a RONA medium comprising:
(i) a basal medium being a combination of KnockOut^{™} DMEM/F12 medium and Neurobasal^{™} medium; and
(ii) an additive comprising a combination of N-2 supplement, B-27^{™} Supplement (XenoFree, minus vitamin A), and GlutaMAX^{™}-I supplement.

28. The use according to any one of claims 13 to 27, wherein the basal medium of said NPC medium is Neurobasal^{™} medium, and the additive of said NPC medium comprises (a) GlutaMAX^{™}-I supplement and (b) B-27^{™} Supplement minus vitamin A.

29. The use according to claim 28, wherein said NPC medium further comprises one or more selected from the group consisting of: brain-derived neurotrophic factor (BDNF), glial cell line-derived neurotrophic factor (GDNF), L-ascorbic acid, and sodium N6,2'-O-dibutyryladenosine 3',5'-cyclic monophosphate (DB-cAMP).

30. The use according to any one of claims 12 to 29, wherein said step (5) employs an NPC medium comprising:
(i) a basal medium being Neurobasal^{™} medium;
(ii) an additive comprising a combination of B-27^{™} Supplement (XenoFree, minus vitamin A) and GlutaMAX^{™}-I supplement; and
(iii) BDNF, GDNF, L-ascorbic acid, and DB-cAMP.

31. The use according to any one of claims 13 to 30, wherein said step (4) uses said RONA medium or said NPC medium.

32. The use according to any one of claims 1 to 31, wherein the pluripotent stem cells in said cell population account for ≤3%, preferably ≤2%, more preferably ≤1%, of the total cell types.

33. The use according to any one of claims 1 to 32, wherein said human forebrain neural progenitor cells are capable of differentiating into neuronal cells.

34. The use according to any one of claims 1 to 33, wherein said human forebrain neural progenitor cells are capable of differentiating into astrocytes.

35. The use according to claim 33, wherein said neuronal cells express MAP2.

36. The use according to claim 34, wherein said astrocytes express GFAP and/or S100β.

37. The use according to any one of claims 1 to 36, wherein said cell population is capable of establishing new neural circuits in a subject.

38. The use according to any one of claims 1 to 37, wherein said disease is a neurotraumatic disease selected from the group consisting of: diseases associated with ischemic brain injury, diseases associated with hemorrhagic brain injury, and diseases associated with traumatic brain injury.

39. The use according to claim 38, wherein said disease associated with ischemic brain injury is ischemic stroke or a condition related to ischemic stroke, including complications or sequelae.

40. The use according to claim 38, wherein said disease associated with hemorrhagic brain injury is hemorrhagic stroke or a condition related to hemorrhagic stroke, including complications or sequelae.

41. The use according to any one of claims 1 to 37, wherein said disease is a neurodegenerative disease selected from the group consisting of: Alzheimer's disease (AD), amyotrophic lateral sclerosis (ALS), and Huntington's disease (HD).

42. The use according to any one of claims 1 to 37, wherein said disease is a neurodevelopmental disorder selected from the group consisting of: autism spectrum disorder, epilepsy, and cerebral palsy.

43. The use according to claim 39 or 40, wherein administration of said cell population occurs after the acute phase of the stroke or at least 14 days post-stroke.

44. The use according to any one of claims 1 to 43, wherein said cell population is administered via intracranial injection.

45. The use according to claim 44, wherein said cell population is injected into the infarct core, the damaged brain region, or into the peri-infarct motor cortex and/or basal ganglia regions adjacent to the infarct or damaged area.

46. The use according to claim 44 or 45, wherein said injection is a single-site or multi-site injection.

47. The use according to any one of claims 44 to 46, wherein said injection is administered once or multiple times.

48. The use according to claim 47, wherein for multiple injections, the interval between consecutive administrations is at least 30 days.

49. The use according to any one of claims 1 to 48, wherein said cell population is formulated as a suspension.

50. The use according to claim 49, wherein said suspension is prepared with 0.9% sodium chloride injection.

51. The use according to claim 49 or 50, wherein the cell concentration in said suspension is 1.0×10⁴ to 5.0×10⁵ viable cells/µL.

52. The use according to any one of claims 44 to 48, wherein the injection dose is 1.0×10⁵ to 1×10¹⁰ cells per dose, preferably 1.5×10⁵ to 6×10⁷ cells per dose.

53. The use according to any one of claims 44 to 48, wherein the subject is a human, and the injection dose is 2×10⁵ to 2×10⁹ cells, preferably 2×10⁶ to 1.2×10⁹ cells.

54. The use according to any one of claims 1 to 53, wherein said cell population or cell preparation is administered alone or in combination with other drugs or therapies.

55. A method for treating a neurotraumatic disease, neurodegenerative disease, neurodevelopmental disorder, or other neurological disease, disorder, or condition associated with death and/or dysfunction of forebrain neural cells in a subject, comprising administering to said subject the cell population of the present invention, wherein the cell population comprises human forebrain neural progenitor cells.

56. The method according to claim 55, wherein said cell population is the cell population according to any one of claims 2 to 37.

57. The method according to claim 55 or 56, wherein said disease is according to any one of claims 38 to 42.

58. The method according to any one of claims 55 to 57, wherein said cell population is administered according to any one of claims 44 to 52.
